# EUROPÄISCHE PATENTSCHRIFT

(11) **EP 2 102 309 B1**
(45) Veröffentlichungstag und Bekanntmachung des Hinweises auf die Patenterteilung: **30.06.2010**
(21) Anmeldenummer: 07856516.5
(22) Anmeldetag: 10.12.2007
(51) Int. Cl.: C09K 11/06, H05B 33/14

(54) **CARBAZOL-DERIVATE FÜR ORGANISCHE ELEKTROLUMINESZENZVORRICHTUNGEN**
CARBAZOLE DERIVATIVES FOR ORGANIC ELECTROLUMINESCENT DEVICES
DÉRIVÉS DE CARBAZOLE POUR DES DISPOSITIFS ÉLECTROLUMINESCENTS ORGANIQUES

(30) Priorität: 18.01.2007 DE 102007002714
(43) Veröffentlichungstag der Anmeldung: 23.09.2009
(73) Patentinhaber: Merck Patent GmbH, 64293 Darmstadt (DE)
(72) Erfinder: PARHAM, Amir, Hossain, 65929 Frankfurt (DE); KROEBER, Jonas, Valentin, 60311 Frankfurt (DE); BUESING, Arne, 65929 Frankfurt am Main (DE); VESTWEBER, Horst, 34630 Gilersberg-Winterscheid (DE)
(86) Internationale Anmeldenummer: PCT/EP2007/010742
(87) Internationale Veröffentlichungsnummer: WO 2008/086851

(56) Entgegenhaltungen:
- EP-A- 1 589 787
- EP-A- 1 708 547
- WO-A-2005/016882
- US-A1- 2003 205 696
- US-B1- 6 670 054

## Beschreibung

Organische Halbleiter werden für eine Reihe verschiedenartiger Anwendungen, die im weitesten Sinne der Elektronikindustrie zugerechnet werden können, entwickelt. Der Aufbau organischer Elektrolumineszenzvorrichtungen (OLEDs), in denen diese organischen Halbleiter als funktionelle Materialien eingesetzt werden, ist beispielsweise in US 4539507, US 5151629, EP 0676461 und WO 98/27136 beschrieben.

Eine Entwicklung der letzten Jahre ist der Einsatz metallorganischer Komplexe, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Ob sich diese Entwicklung durchsetzen wird, hängt davon ab, ob entsprechende Device-Kompositionen gefunden werden, die diese Vorteile (Triplett-Emission = Phosphoreszenz gegenüber Singulett-Emission = Fluoreszenz) auch in den OLEDs umsetzen können.

Generell gibt es bei OLEDs, die Triplett-Emission zeigen, immer noch erhebliche Probleme. So ist die operative Lebensdauer allgemein zu gering, was bislang noch der Einführung von phosphoreszierenden OLEDs in hochwertigen und langlebigen Vorrichtungen entgegensteht. In phosphoreszierenden OLEDs wird als Matrixmaterial häufig 4,4'-Bis-(N-carbazolyl)biphenyl (CBP) verwendet. Die Nachteile sind kurze Lebensdauern der damit hergestellten Devices und hohe Betriebsspannungen, die zu geringen Leistungseffizienzen führen. Außerdem weist CBP eine nicht ausreichend hohe Glasübergangstemperatur auf. Trotz aller Nachteile, die CBP aufweist, wird dieses weiterhin als Triplett-Matrixmaterial verwendet, da auch mit alternativen Matrixmaterialien die oben beschriebenen Probleme noch nicht zufrieden stellend gelöst werden.

Aufgabe der vorliegenden Erfindung ist daher die Bereitstellung von Carbazolderivaten, welche die oben aufgeführten Probleme nicht aufweisen und welche insbesondere eine höhere Glasübergangstemperatur aufweisen, ohne dass sich dadurch die anderen Deviceeigenschaften negativ verändern. Weiterhin ist Aufgabe der vorliegenden Erfindung die Bereitstellung von Carbazolderivaten, welche bei Verwendung als Triplett-Matrixmaterial in OLEDs zu verbesserten Effizienzen und Lebensdauern führen.

Überraschend wurde gefunden, dass Derivate von CBP und andere Carbazolderivate, in welchen das Carbazol in 2-Position durch eine aromatische oder heteroaromatische Gruppe substituiert ist, hier deutliche Verbesserungen zeigt. Insbesondere führt dies zu Derivaten mit deutlich erhöhter Glasübergangstemperatur und im Device zu längeren Lebensdauern und höheren Effizienzen, ohne dass sich die anderen elektronischen Eigenschaften der Verbindung negativ verändern. Diese Materialien und deren Verwendung in organischen elektronischen Vorrichtungen sind daher der Gegenstand der vorliegenden Erfindung.

US 6,562,982 offenbart Derivate von CBP, welche in 3,6-Position mit Arylgruppen substituiert sind, als Ladungstransportverbindungen für organische Elektrolumineszenzvorrichtungen. Die Glasübergangstemperaturen dieser Verbindungen sind nicht angegeben. Allerdings stehen in diesen Verbindungen die Arylsubstituenten in Konjugation mit dem Stickstoff des Carbazols und haben damit einen deutlichen Einfluss auf die elektronischen Eigenschaften der Verbindung. Es ist daher so nicht möglich, CBP-Derivate mit vergleichbaren elektronischen Eigenschaften wie CBP zu erhalten.

In JP 2004/288381 werden Carbazolderivate als Triplett-Matrixmaterialien offenbart, welche mit fluorierten Aromaten substituiert sind. Dabei sind die fluorierten Arylsubstituenten in der 2- oder in der 3-Position am Carbazol gebunden. Durch die hohe Elektronegativität des Fluors haben diese Substituenten jedoch einen starken Einfluss auf die elektronischen Eigenschaften des Moleküls.

Gegenstand der Erfindung sind Verbindungen gemäß Formel (1), 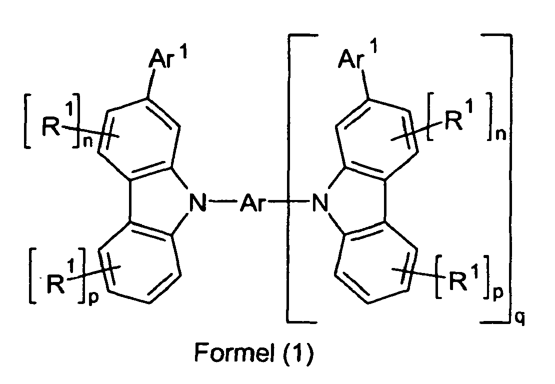 wobei für die verwendeten Symbole und Indizes gilt:
- Ar: ist bei jedem Auftreten ein aromatisches oder heteroaromatisches Ringsystem mit 5 bis 60 aromatischen Ringatomen, welches mit einem oder mehreren Resten R¹ substituiert sein kann;
- Ar¹: ist bei jedem Auftreten gleich oder verschieden ein aromatisches oder heteroaromatisches Ringsystem mit 5 bis 60 aromatischen Ringatomen, welches mit einem oder mehreren Resten R substituiert sein kann;
- R: ist bei jedem Auftreten gleich oder verschieden Cl, Br, I, N(Ar²)₂, CN, NO₂, Si(R²)₃, B(OR²)₂, C(=O)Ar², P(=O)(Ar²)₂, S(=O)Ar², S(=O)₂Ar², -CR²=CR²(Ar²), OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ring- system mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Kombination dieser Systeme; dabei können zwei oder mehrere Substituenten R auch miteinander ein mono- oder polycyclisches aliphatisches oder aromatisches Ringsystem bilden;
- R¹: ist bei jedem Auftreten gleich oder verschieden R, eine Gruppe Ar¹ oder F;
- Ar²: ist bei jedem Auftreten gleich oder verschieden ein aromatisches oder heteroaromatisches Ringsystem mit 5 bis 60 aromatischen Ringatomen, welches mit einem oder mehreren Resten R² substituiert sein kann;
- R²: ist bei jedem Auftreten gleich oder verschieden H oder ein aliphatischer, aromatischer und/oder heteroaromatischer Kohlen- wasserstoffrest mit 1 bis 20 C-Atomen; dabei können zwei oder mehrere Substituenten R² auch miteinander ein mono- oder poly- cyclisches aliphatisches oder aromatisches Ringsystem bilden;
- n: ist bei jedem Auftreten gleich oder verschieden 0, 1, 2 oder 3;
- p: ist bei jedem Auftreten gleich oder verschieden 0, 1, 2, 3 oder 4;
- q: ist 1, 2, 3, 4 oder 5.

Wenn der Index q gleich 1 ist, bedeutet dies, dass Ar eine bivalente Gruppe darstellt. Wenn der Index q größer als 1 ist, bedeutet dies, dass insgesamt drei oder mehr Carbazolgruppen an das aromatische Ringsystem Ar gebunden sind. Ar ist für q = 2 eine trivalente Gruppe und für q > 2 eine entsprechend höhervalente Gruppe. Bevorzugt ist der Index q = 1 oder 2, besonders bevorzugt ist q = 1.

Bevorzugt weisen die erfindungsgemäßen Verbindungen eine Glasübergangstemperatur T_{g} von mehr als 120 °C auf, besonders bevorzugt mehr als 140 °C.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Das aromatische Ringsystem enthält bevorzugt keine Metallatome.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 60 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol. In einer bevorzugten Ausführungsform der Erfindung sind die Indizes n in Verbindungen der Formel (1) bei jedem Auftreten gleich oder verschieden 0 oder 1. Besonders bevorzugt sind die Indizes n = 0.

Bevorzugte Strukturen gemäß Formel (1) sind die Verbindungen gemäß den Formeln (2) bis (7), 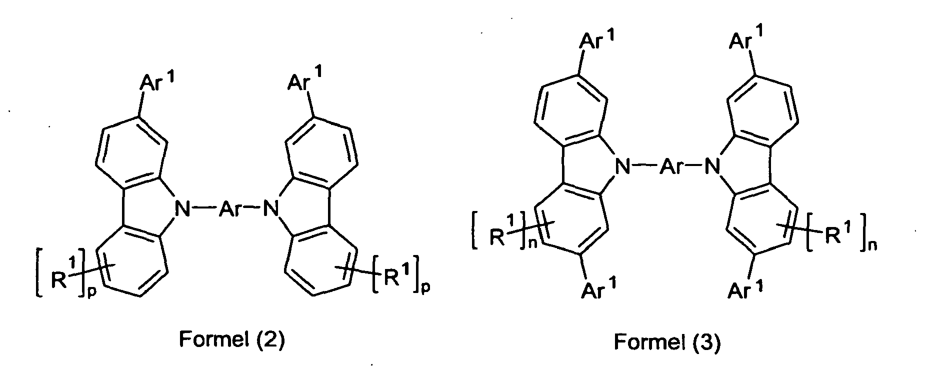 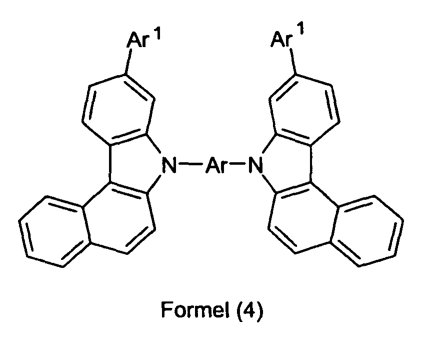 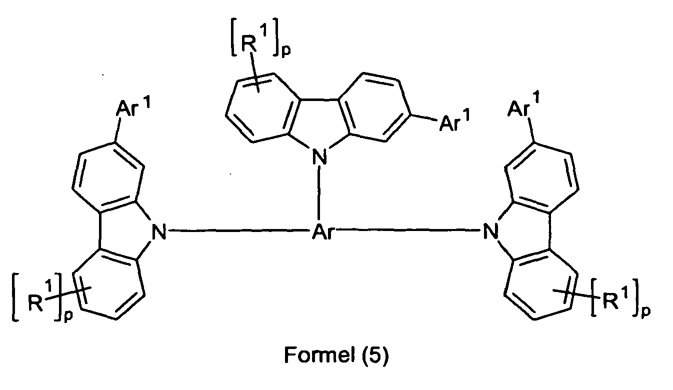 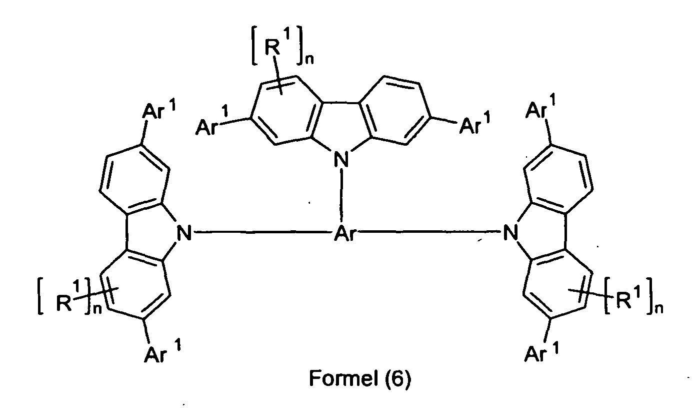 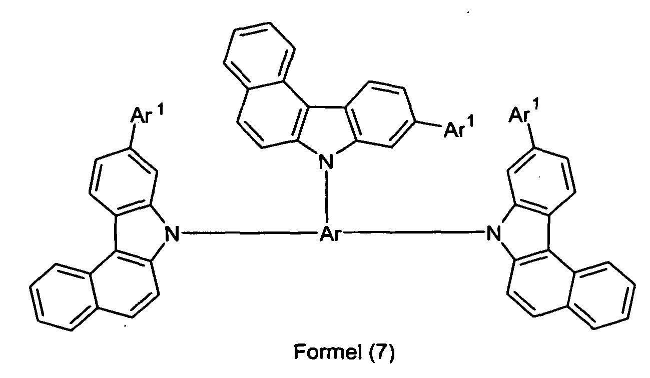 wobei die Symbole und Indizes die oben aufgeführten Bedeutungen haben.

In einer bevorzugten Ausführungsform der Verbindungen gemäß Formel (1), bzw. gemäß Formel (2) oder Formel (5) ist der Index p gleich oder verschieden bei jedem Auftreten 0, 1 oder 2, besonders bevorzugt 0 oder 1. Wenn der Index p gleich 1 ist, ist der Substituent R¹ bevorzugt in der 5-Position oder in der 7-Position des Carbazols gebunden, besonders bevorzugt in der 5-Position. Wenn der Index p gleich 2 ist, sind die Substituenten R¹ bevorzugt in der 5- und in der 7-Position des Carbazols gebunden.

In einer bevorzugten Ausführungsform der Verbindungen gemäß Formel (3) bzw. Formel (6) ist der Index n gleich oder verschieden bei jedem

Auftreten 0 oder 1. Wenn der Index n gleich 1 ist, ist der Substituent R¹ bevorzugt in der 5-Position des Carbazols gebunden.

Der Übersichtlichkeit halber ist in der folgenden Formel die Nummerierung der Positionen des Carbazols dargestellt:

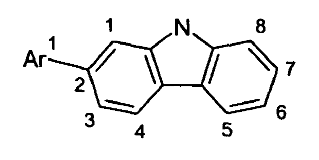

Bevorzugte Gruppen Ar und Ar¹ in Formel (1) bzw. in den Formeln (2) bis (7) enthalten nur Phenyl- und/oder Naphthylgruppen oder heteroaromatische Gruppen mit nicht mehr als zwei kondensierten aromatischen bzw. heteroaromatischen Ringen, jedoch keine größeren kondensierten aromatischen Systeme. Daher sind bevorzugte Gruppen Ar und Ar¹ aromatische Ringsysteme, welche aufgebaut sind aus Phenyl- und/oder Naphthylgruppen oder Verknüpfungen dieser Systeme, wie beispielsweise Biphenyl, Fluoren, Spirobifluoren, etc. Weiterhin bevorzugt als Gruppe Ar bzw. Ar¹ ist Carbazol.

Besonders bevorzugte Gruppen Ar sind gewählt aus 1,2-Phenylen, 1,3-Phenylen, 1,4-Phenylen, 1,3,5-Benzol, 3,3'-Biphenyl, 4,4'-Biphenyl, 1,3,5-Triphenylbenzol, Triphenylamin, 2,7-Fluorenylen, welches durch einen oder mehrere Reste R¹ substituiert sein kann, 2,7-Spirobifluorenylen, welches durch einen oder mehrere Reste R¹ substituiert sein kann, Indenofluorenylen, welches durch einen oder mehrere Reste R¹ substituiert sein kann, 4,4"'-(1,1':2',1",2",1"'-Quarterphenyl), 4,4'-(2,2'-Dimethylbiphenyl), 4,4'-(1,1'-Binaphthyl), 4,4'-Stilbenyl oder Dihydrophenanthrenyl, welches durch einen oder mehrere Reste R¹ substituiert sein kann.

Besonders bevorzugte Gruppen Ar¹ sind gleich oder verschieden gewählt aus Phenyl, 1-Naphthyl, 2-Naphthyl, 2-Carbazolyl, 3-Carbazolyl, 9-Carbazolyl, Triphenylamin, Naphthyldiphenylamin oder Dinaphthylphenylamin, welche jeweils durch einen oder mehrere Reste R substituiert sein können. Dabei können die beiden letztgenannten Gruppen über das Naphthalin in 1- oder 2-Position oder über die Phenylgruppe gebunden sein. Eine 2- bzw. 3-Carbazolylgruppe ist dabei bevorzugt am Stickstoff durch einen aromatischen Rest R substituiert.

Bevorzugt sind weiterhin Verbindungen gemäß Formel (1), bzw. Verbindungen gemäß den Formeln (2) bis (7), in denen das Symbol R, also der Substituent an der Gruppe Ar¹, gleich oder verschieden bei jedem Auftreten für H, N(Ar²)₂, eine geradkettige Alkylgruppe mit 1 bis 5 C-Atomen oder verzweigte Alkylgruppe mit 3 bis 5 C-Atomen, wobei jeweils eine oder mehrere nicht benachbarte CH₂-Gruppen durch -R²C=CR²- oder -O- ersetzt sein können und wobei ein oder mehrere H-Atome durch F ersetzt sein können, oder eine Arylgruppe mit 6 bis 16 C-Atomen oder Heteroarylgruppe mit 2 bis 16 C-Atomen oder eine Spirobifluorengruppe, die jeweils mit einem oder mehreren Resten R² substituiert sein kann, oder eine Kombination aus zwei dieser Systeme steht. Besonders bevorzugte Reste R sind gleich oder verschieden bei jedem Auftreten H, Methyl, Ethyl, iso-Propyl, tert-Butyl, wobei jeweils ein oder mehrere H-Atome durch F ersetzt sein können, oder eine Phenyl-, Naphthyl- oder Spirobifluorenylgruppe, die jeweils mit einem oder mehreren Resten R² substituiert sein kann, oder eine Kombination aus zwei dieser Systeme. Bei Verbindungen, die aus Lösung verarbeitet werden, sind auch lineare oder verzweigte Alkylketten mit bis zu 10 C-Atomen bevorzugt. Brom, Boronsäure bzw. Boronsäurederivate als Substituenten sind vor allem für die Verwendung dieser Verbindung als Zwischenverbindung zur Herstellung weiterer erfindungsgemäßer Verbindungen bevorzugt.

Bevorzugt sind weiterhin Verbindungen gemäß Formel (1), bzw. Verbindungen gemäß den Formeln (2) bis (7), in denen das Symbol R¹ gleich oder verschieden bei jedem Auftreten entsprechend dem bevorzugten Substituenten R definiert ist oder für Ar¹ oder F steht.

Weiterhin bevorzugt sind symmetrische Verbindungen, also Verbindungen, in denen alle Symbole Ar¹ gleich sind und die gleich substituiert sind. Beispiele für bevorzugte Verbindungen gemäß Formel (1) sind die im Folgenden abgebildeten Verbindungen (1) bis (72).

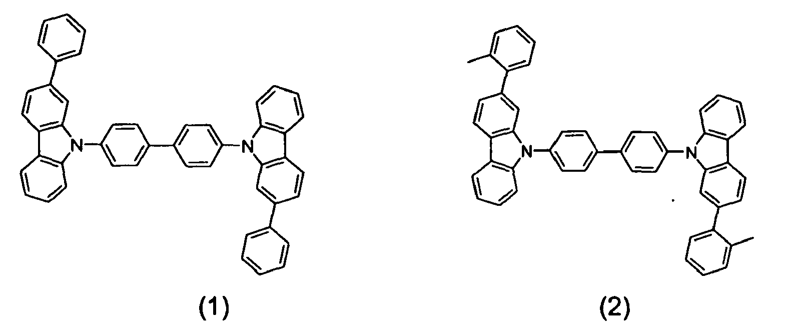

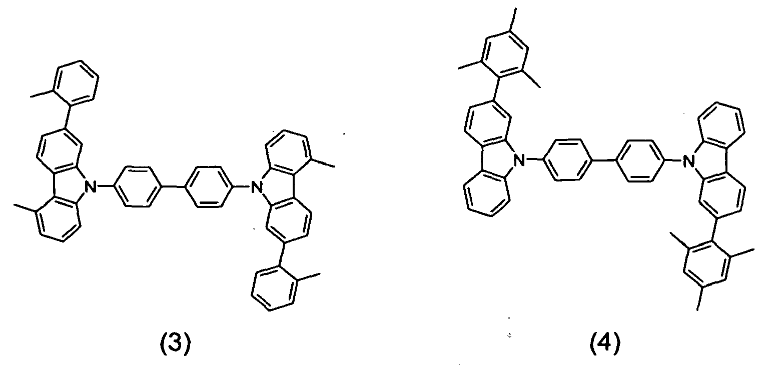

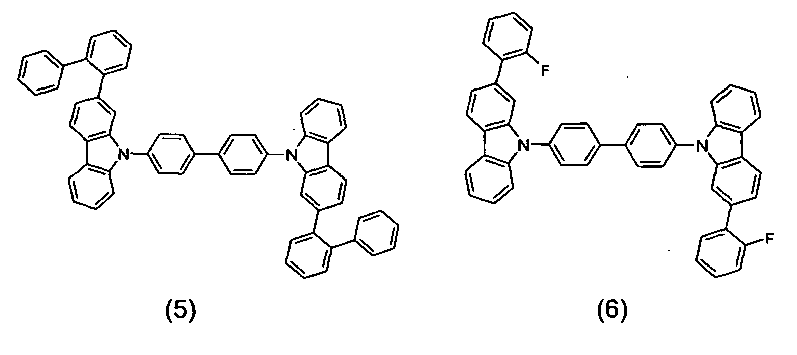

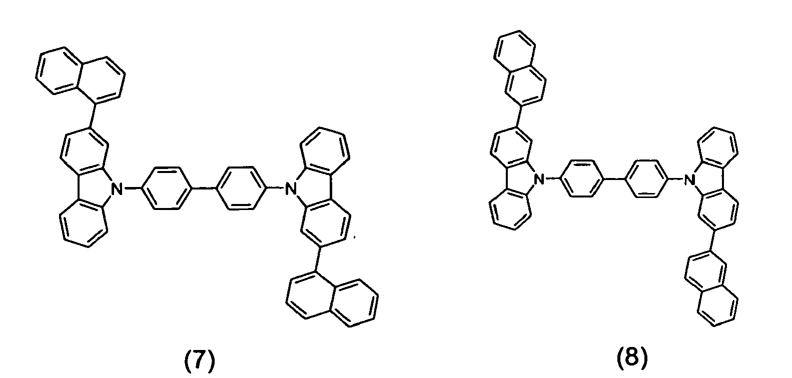

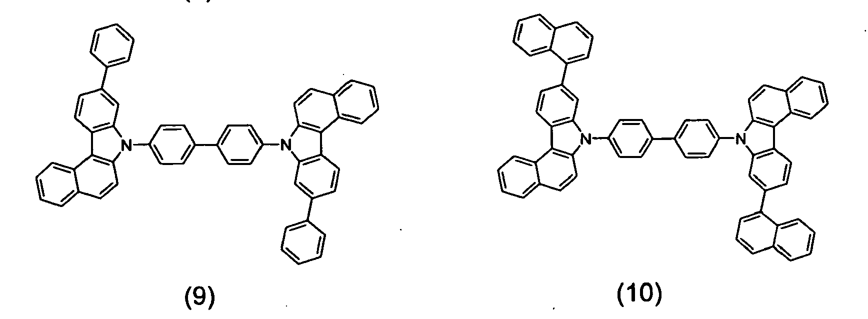

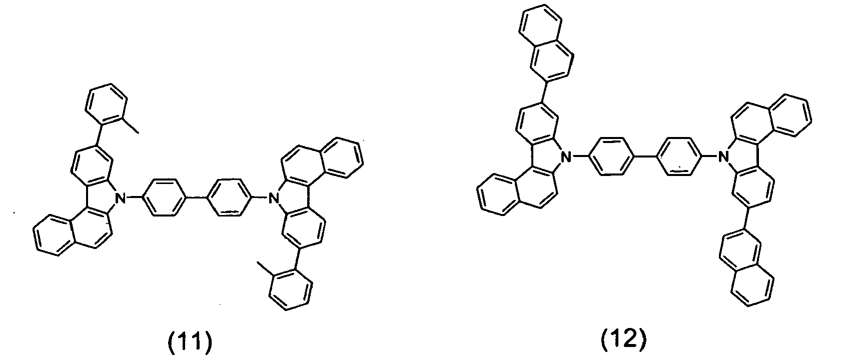

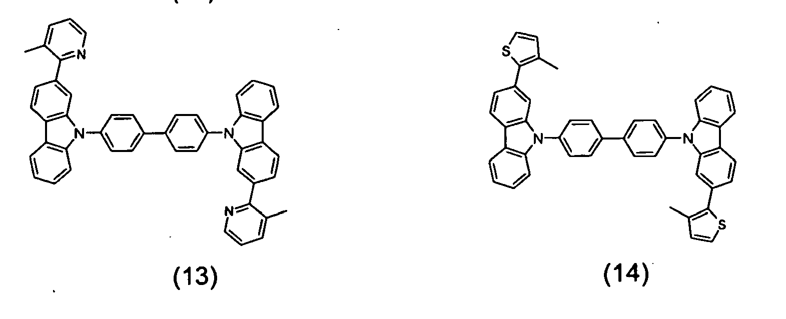

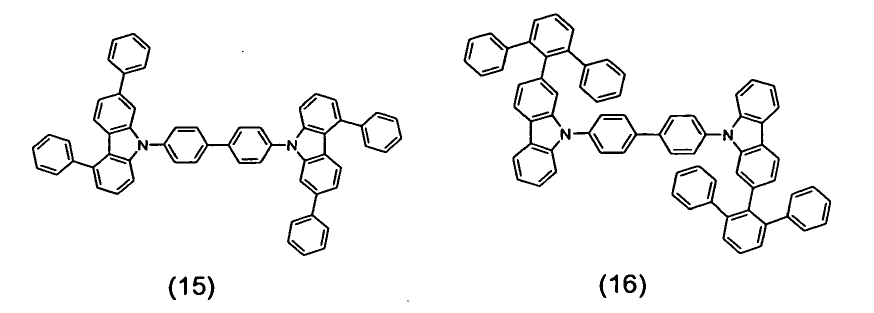

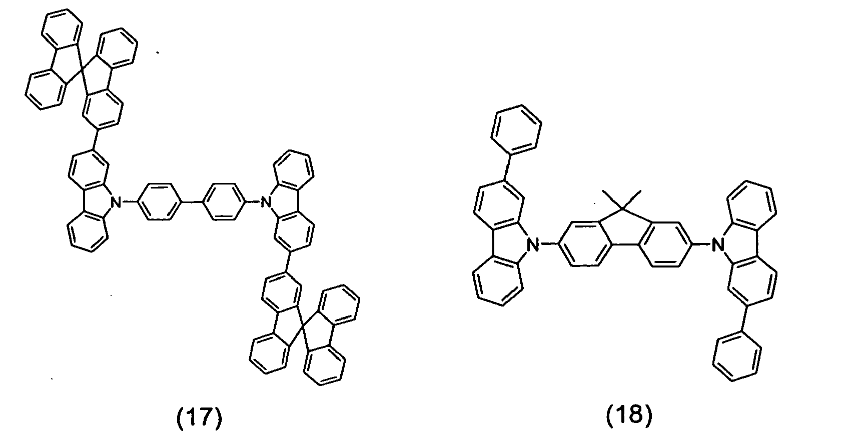

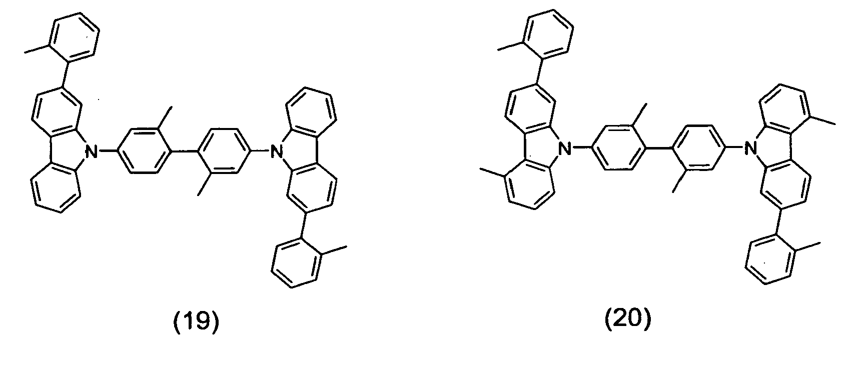

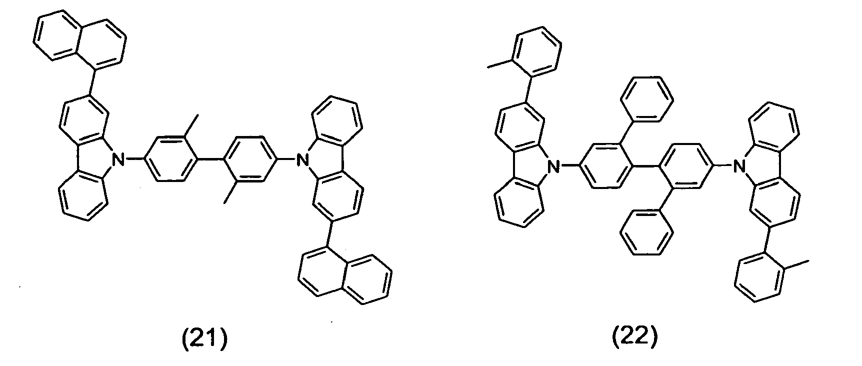

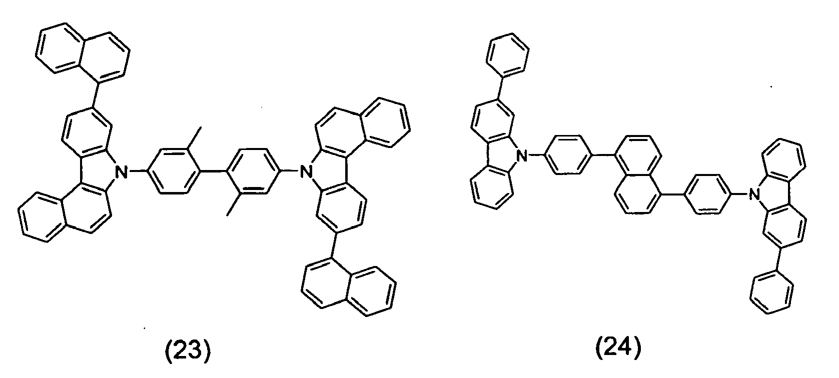

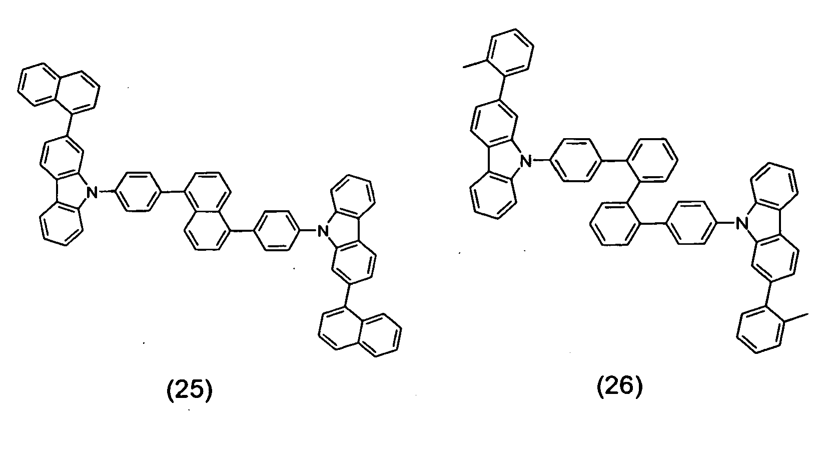

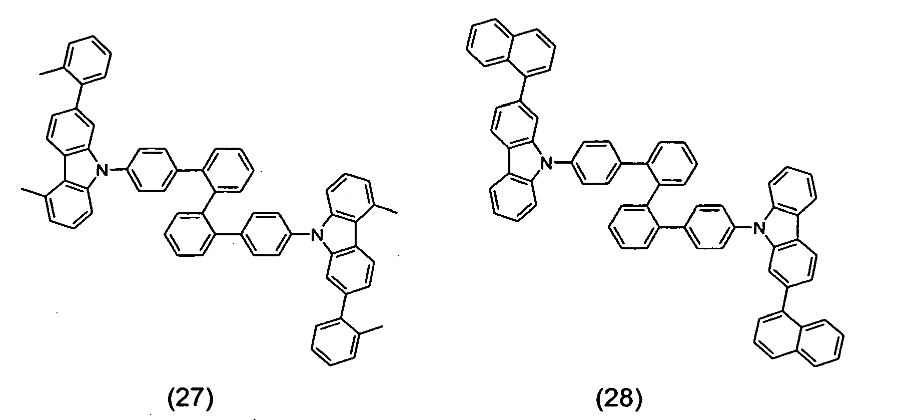

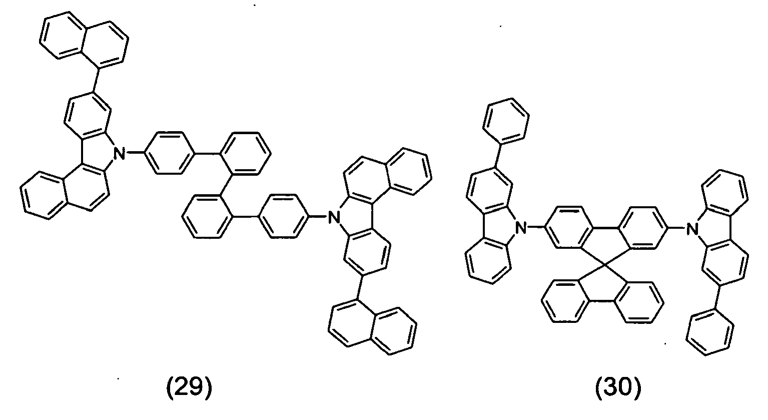

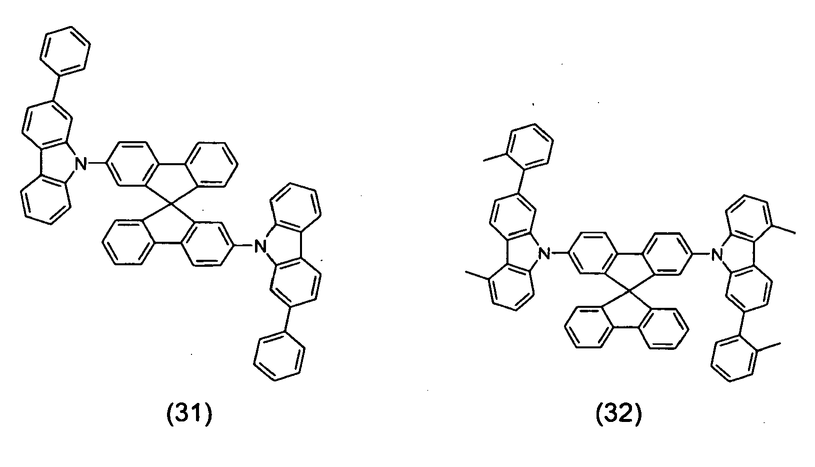

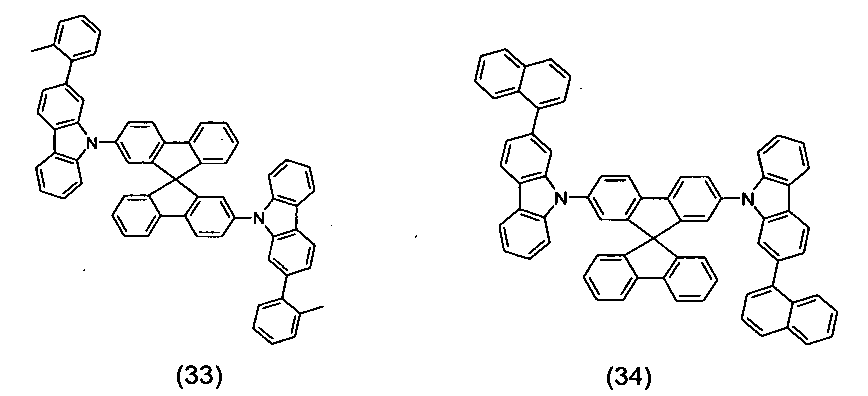

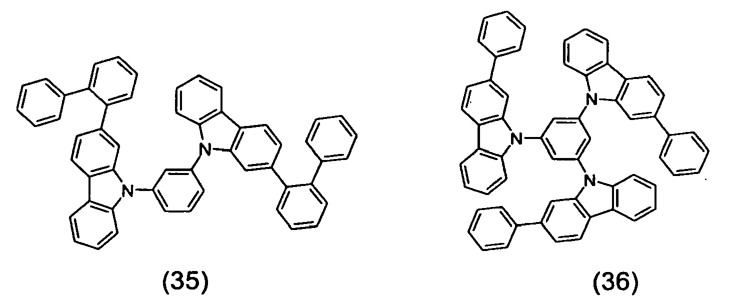

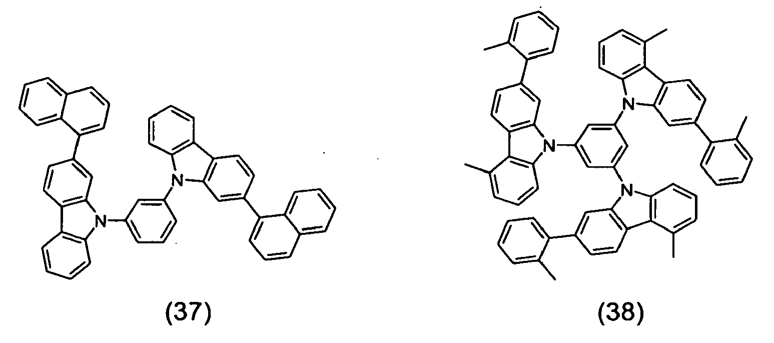

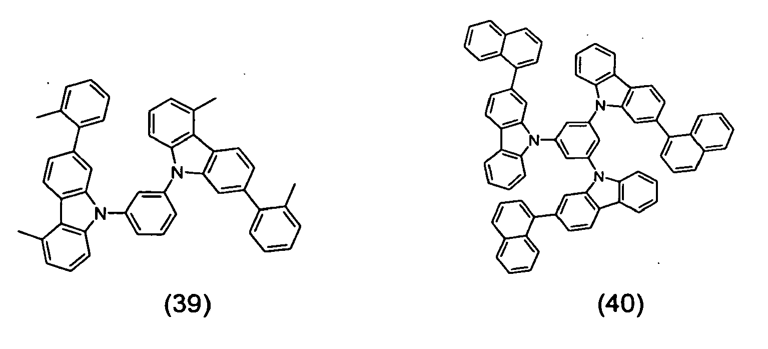

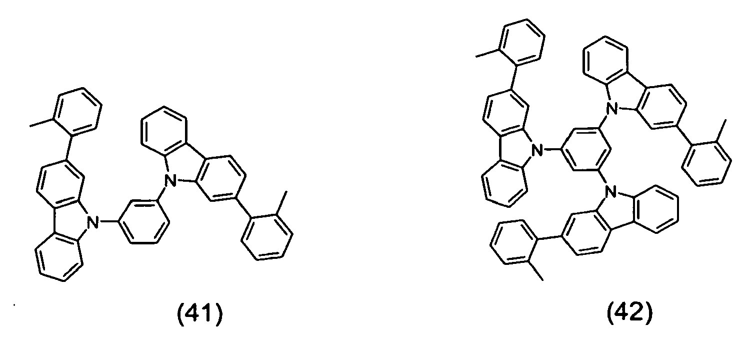

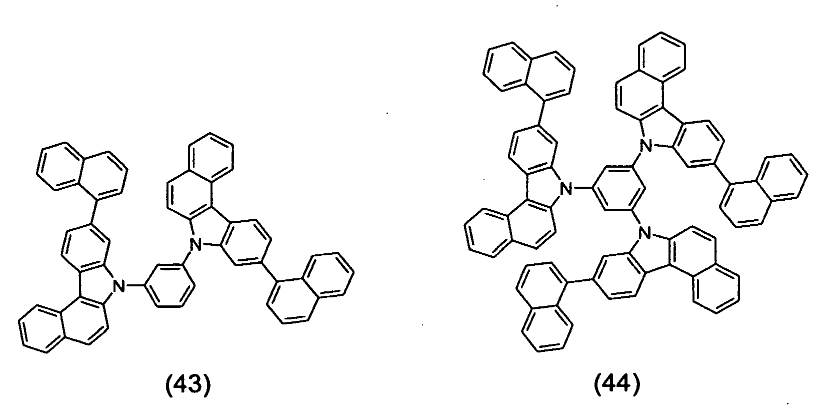

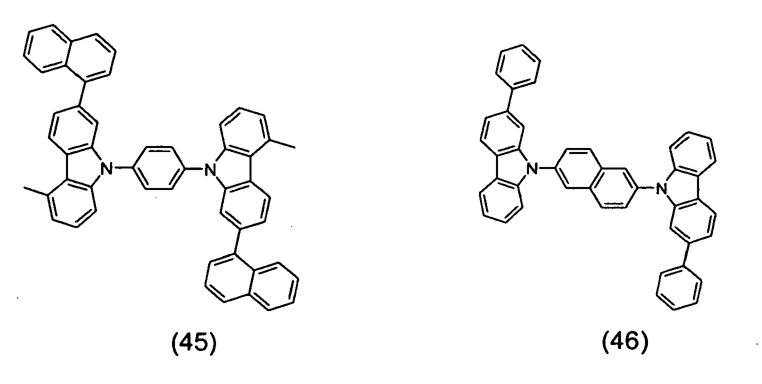

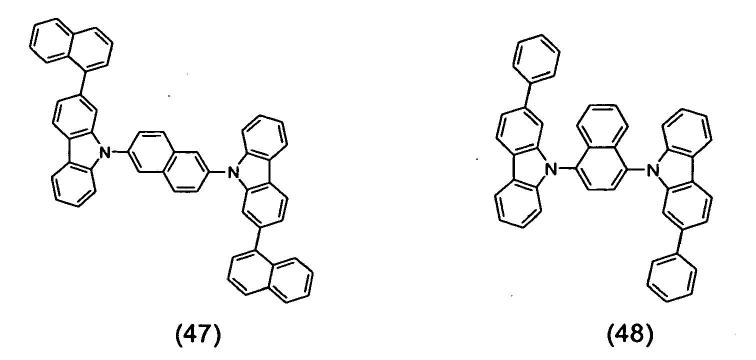

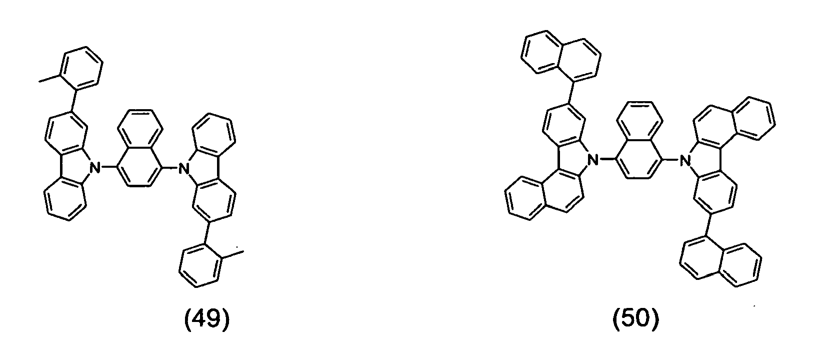

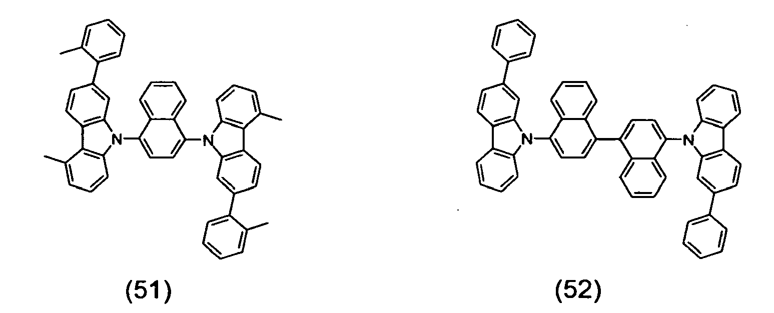

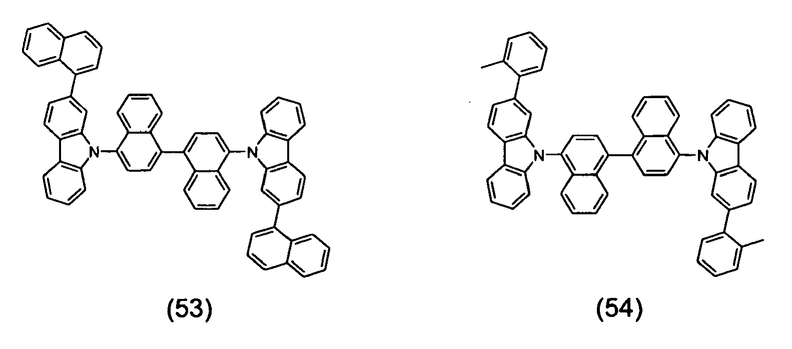

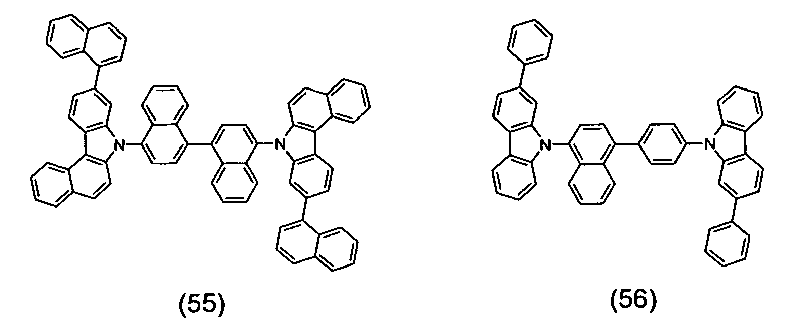

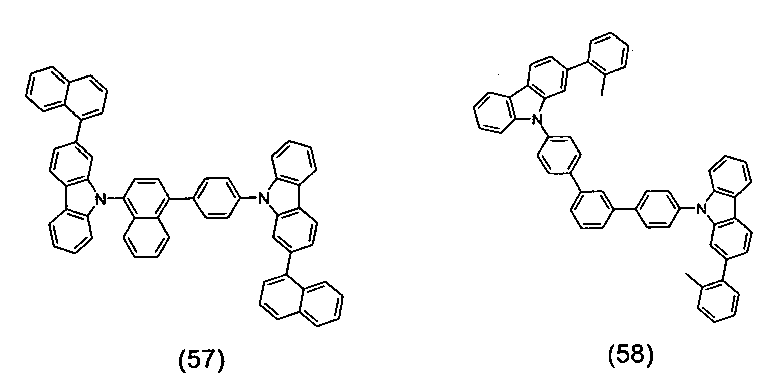

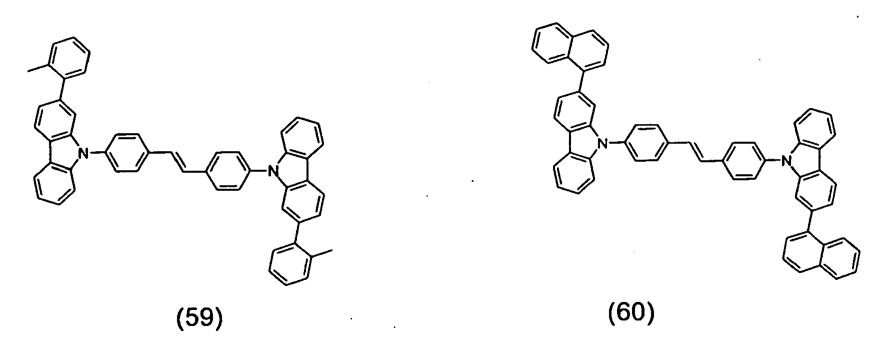

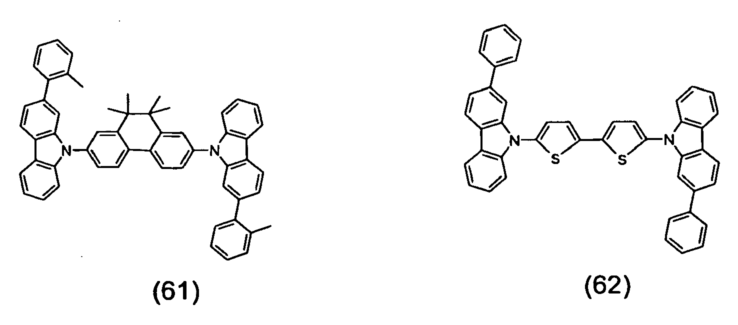

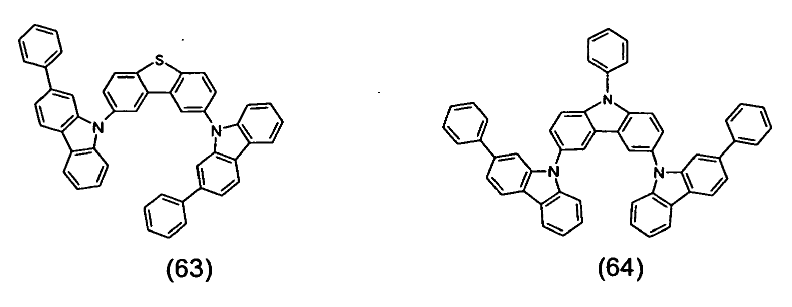

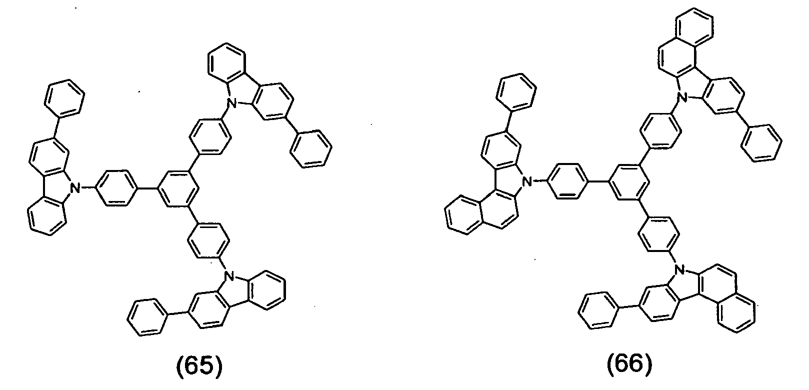

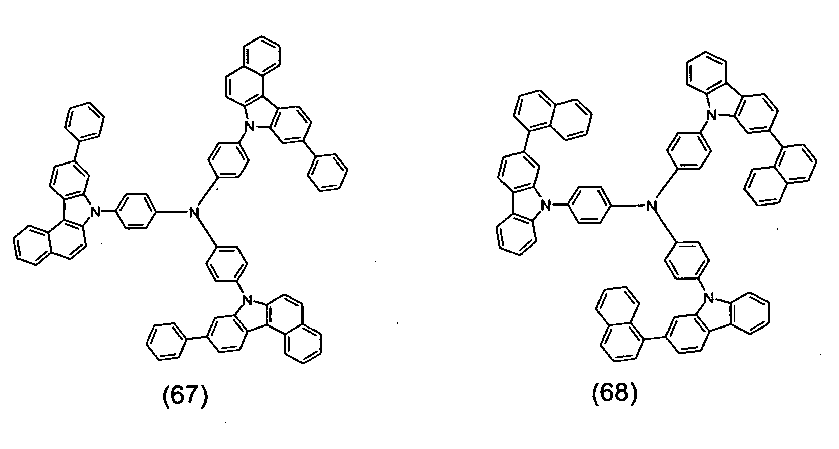

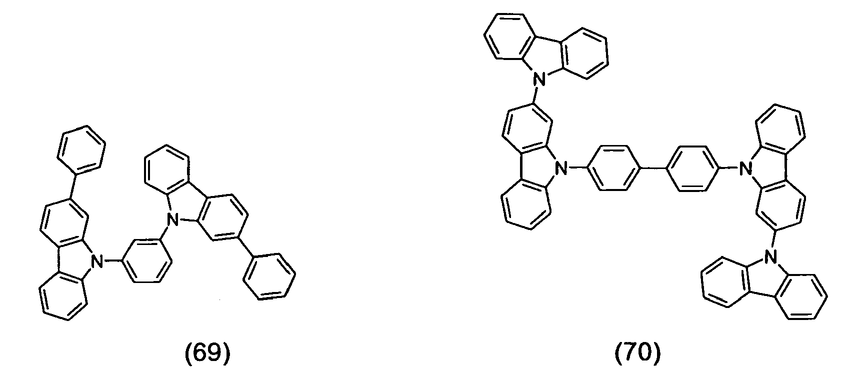

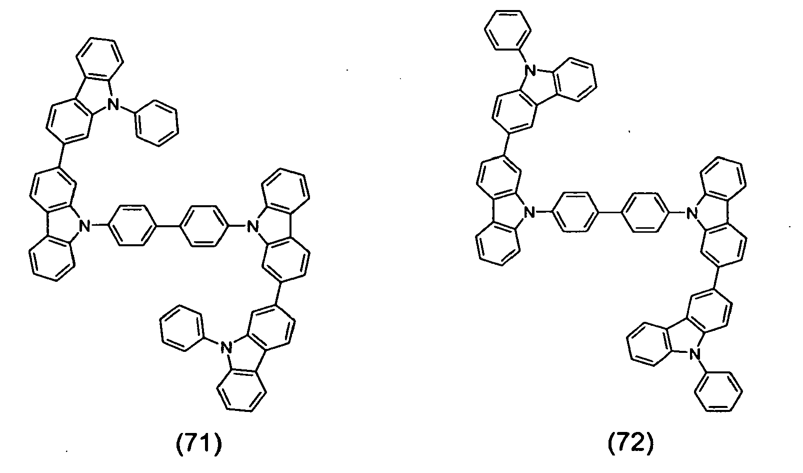

Die erfindungsgemäßen Verbindungen können nach Standardmethoden der organischen Chemie synthetisiert werden. So ist bekannt, dass 2-Nitrobiphenylderivate mit einem Trialkylphosphit zu den entsprechenden Carbazolderivaten umgesetzt werden können (M. Tavasli et al., Synthesis 2005, 1619-1624). Diese Reaktion lässt sich zum Aufbau von 2-Arylsubstituierten Carbazolderivaten verwenden, indem zunächst ein entsprechendes Aryl-substituiertes 2-Nitrobiphenylderivat aufgebaut wird, welches anschließend mit Trialkylphosphit umgesetzt wird. Das 2-Arylsubstituierte Carbazolderivat lässt sich mit einem Dibromaromaten in einer Hartwig-Buchwald-Kupplung unter Standardbedingungen zur Verbindung gemäß Formel (1) kuppeln. Die verschiedenen Ausführungsmethoden und Reaktionsbedingungen der Hartwig-Buchwald-Kupplung sind dem Fachmann der organischen Synthese bekannt. Statt eines Dibromaromaten lassen sich auch entsprechende Verbindungen mit anderen Abgangsgruppen, beispielsweise Chlor, Iod, Triflat, Tosylat oder allgemein Sulfonate, verwenden. Durch Verwendung von trisubstituierten Aromaten oder Verbindungen mit nochmals mehr Abgangsgruppen lassen sich entsprechend Verbindungen gemäß Formel (1) synthetisieren, in denen der Index q für 2 oder mehr steht.

Die Synthese von Verbindungen gemäß Formel (1) ist im folgenden Schema 1 abgebildet, wobei der Übersichtlichkeit halber q = 1 gewählt wurde und keine Substituenten R oder R¹ abgebildet sind:

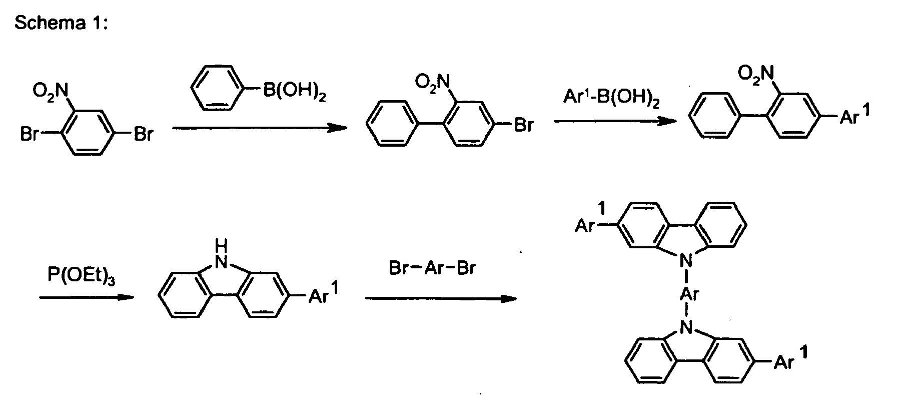

Ein weiterer Gegenstand der vorliegenden Erfindung ist ein Verfahren zur Herstellung von Verbindungen gemäß Formel (1), ausgehend von einem 4-Aryl-2-nitro-1,1'-biphenyl oder 4-Heteroaryl-2-nitro-1,1'-biphenyl, wobei die Arylgruppe bzw. Heteroarylgruppe auch durch einen oder mehrere Reste R substituiert sein kann und das Biphenyl auch durch einen oder mehrere Reste R¹ substituiert sein kann, welches mit einem Trialkylphosphit, wobei die Alkylgruppen gleich oder verschieden bei jedem Auftreten 1 bis 10 C-Atome aufweisen, zum entsprechenden Carbazol umgesetzt wird, gefolgt von einer Hartwig-Buchwald-Kupplung mit einer aromatischen Verbindung, welche mindestens zwei reaktive Gruppen aufweist. Die reaktiven Gruppen für die Hartwig-Buchwald-Kupplung sind bevorzugt gewählt aus Chlor, Brom, Iod, Triflat, Tosylat oder OSO₂-R², wobei R² dieselbe Bedeutung hat, wie oben ausgeführt.

Die erfindungsgemäßen Verbindungen eignen sich für den Einsatz in organischen Elektrolumineszenzvorrichtungen (OLEDs, PLEDs), insbesondere als Triplett-Matrixmaterialien in phosphoreszierenden OLEDs, aber auch als Lochtransportmaterialien.

Ein weiterer Gegenstand der Erfindung ist daher die Verwendung von Verbindungen gemäß Formel (1) in organischen elektronischen Vorrichtungen, insbesondere in organischen Elektrolumineszenzvorrichtungen.

Nochmals ein weiterer Gegenstand der Erfindung sind organische elektronische Vorrichtungen, enthaltend mindestens eine Verbindung gemäß Formel (1), insbesondere organische Elektrolumineszenzvorrichtungen, enthaltend Anode, Kathode und mindestens eine emittierende Schicht, dadurch gekennzeichnet, dass mindestens eine Schicht mindestens eine Verbindung gemäß Formel (1) enthält.

Außer Kathode, Anode und der emittierenden Schicht kann die organische Elektrolumineszenzvorrichtung noch weitere Schichten enthalten, beispielsweise jeweils eine oder mehrere Lochinjektionsschichten, Lochtransportschichten, Lochblockierschichten, Elektronentransportschichten, Elektroneninjektionsschichten und/oder Ladungserzeugungsschichten (Charge-Generation Layers, IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer)*.* Ebenso können zwischen zwei emittierende Schichten Interlayers eingebracht sein, welche beispielsweise eine Excitonen-blockierende Funktion aufweisen. Es sei aber darauf hingewiesen, dass nicht notwendigerweise jede dieser Schichten vorhanden sein muss.

In einer weiteren bevorzugten Ausführungsform der Erfindung enthält die organische Elektrolumineszenzvorrichtung mehrere emittierende Schichten, wobei mindestens eine Schicht mindestens eine erfindungsgemäße Verbindung enthält. Besonders bevorzugt weisen die Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei mindestens eine dieser Schichten mindestens eine erfindungsgemäße Verbindung enthält und wobei die drei Schichten blaue, grüne und orange oder rote Emission zeigen (für den prinzipiellen Aufbau siehe z. B. WO 05/011013). Ebenso eignen sich für weiße Emission Emitter, welche breitbandige Emissionsbanden aufweisen und dadurch weiße Emission zeigen.

In einer bevorzugten Ausführungsform der Erfindung werden die erfindungsgemäßen Verbindungen als Matrix für phosphoreszierende Dotanden eingesetzt. Unter Phosphoreszenz im Sinne dieser Erfindung wird dabei die Lumineszenz aus einem angeregten Zustand höherer Spinmultiplizität verstanden, insbesondere die Luminesz aus einem angeregten Triplettzustand. Die phosphoreszierenden Dotanden enthalten mindestens eine Verbindung, die bei geeigneter Anregung Licht, vorzugsweise im sichtbaren Bereich, emittiert und außerdem mindestens ein Atom der Ordnungszahl größer 20, bevorzugt größer 38 und kleiner 84, besonders bevorzugt größer 56 und kleiner 80 enthält. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Derartige Emitter sind dem Fachmann auf dem Gebiet der Elektrolumineszenz bekannt.

Besonders bevorzugte organische Elektrolumineszenzvorrichtungen enthalten als phosphoreszierenden Emitter mindestens eine Verbindung der Formeln (8) bis (11), 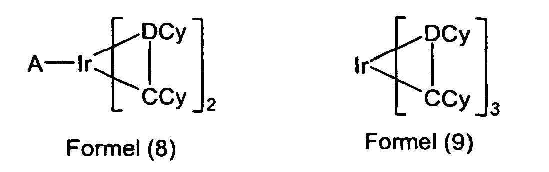 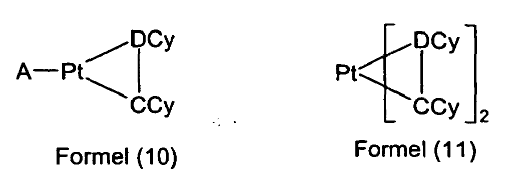 wobei für die verwendeten Symbole gilt:
- DCy: ist gleich oder verschieden bei jedem Auftreten eine cyclische Gruppe, die mindestens ein Donoratom, bevorzugt Stickstoff oder Phosphor, enthält, über welches die cyclische Gruppe an das Metall gebunden ist, und die wiederum einen oder mehrere Substituenten R¹ tragen kann; die Gruppen DCy und CCy sind über eine kovalente Bindung miteinander verbunden;
- CCy: ist gleich oder verschieden bei jedem Auftreten eine cyclische Gruppe, die ein Kohlenstoffatom enthält, über welches die cyclische Gruppe an das Metall gebunden ist und die wiederum einen oder mehrere Substituenten R¹ tragen kann;
- A: ist gleich oder verschieden bei jedem Auftreten ein mono- anionischer, zweizähnig chelatisierender Ligand, bevorzugt ein Diketonatligand;
- R¹: hat dieselbe Bedeutung, wie oben beschrieben.

Dabei kann durch Bildung von Ringsystemen zwischen mehreren Resten R¹ auch eine Brücke zwischen den Gruppen DCy und CCy vorliegen.

Beispiele der oben beschriebenen Emitter können den Anmeldungen WO 00/70655, WO 01/41512, WO 02/02714, WO 02/15645, EP 1191613, EP 1191612, EP 1191614 und WO 05/033244 entnommen werden. Generell eignen sich phosphoreszierende Komplexe, wie sie gemäß dem Stand der Technik für phosphoreszierende OLEDs verwendet werden und wie sie dem Fachmann generell bekannt sind.

Die erfindungsgemäße Mischung enthält zwischen 1 und 99 Gew.-%, vorzugsweise zwischen 2 und 90 Gew.-%, besonders bevorzugt zwischen 3 und 40 Gew.-%, insbesondere zwischen 5 und 15 Gew.-% des phosphoreszierenden Emitters bezogen auf die Gesamtmischung aus Emitter und Matrixmaterialien. Entsprechend enthält die erfindungsgemäße Mischung zwischen 99 und 1 Gew.-%, vorzugsweise zwischen 98 und 10 Gew.-%, besonders bevorzugt zwischen 97 und 60 Gew.-%, insbesondere zwischen 95 und 85 Gew.-% des Matrixmaterials bzw. der Matrixmaterialien bezogen auf die Gesamtmischung aus Emitter und Matrixmaterialien.

Die Verbindung der Formel (1) kann entweder das einzige Matrixmaterial in der emittierenden Schicht sein. Es ist jedoch auch möglich, eine Mischung aus mehreren Matrixmaterialien in der emittierenden Schicht zu verwenden. Dies können mehrere verschiedene Matrixmaterialien gemäß Formel (1) sein. Weiterhin hat es sich als bevorzugt erwiesen, ein Matrixmaterial gemäß Formel (1) zusammen mit einem aromatischen Keton oder einem aromatischen Phosphinoxid, einem aromatischen Sulfoxid oder einem aromatischen Sulfon als weiterem Matrixmaterial und einem phosphoreszierenden Dotanden in der emittierenden Schicht einzusetzen. Bevorzugte aromatische Ketone sind solche, in denen an die Ketogruppe zwei aromatische oder heteroaromatische Ringsysteme gebunden sind. Bevorzugte aromatische Phosphinoxide sind solche, in denen an die Phosphinoxidgruppe drei aromatische oder heteroaromatische Ringsysteme gebunden sind. Insbesondere bevorzugt sind Ketone bzw. Phosphinoxide der folgenden Formeln (12) und (13), 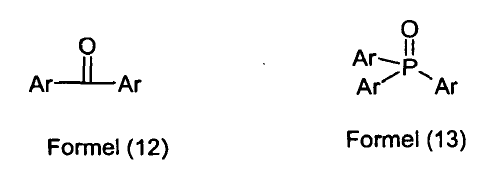 wobei Ar dieselbe Bedeutung hat, wie oben beschrieben.

Besonders geeignete Ketone sind in der Anmeldung WO 04/093207 offenbart. Besonders geeignete Phosphinoxide, Sulfoxide und Sulfone sind in der Anmeldung WO 05/003253 offenbart. Diese Verbindungen können besonders gut zusammen mit den Verbindungen gemäß Formel (1) als Matrixmaterial für phosphoreszierende Emitter eingesetzt werden.

Wenn die Verbindung gemäß Formel (1) zusammen mit einem Keton, einem Phosphinoxid, einem Sulfoxid oder einem Sulfon als Matrixmaterial eingesetzt wird, dann liegt das Verhältnis der Verbindung gemäß Formel (1) zu dem Keton, Phosphinoxid, Sulfoxid bzw. Sulfon bevorzugt im Bereich von 10 :1 bis 1 : 10, besonders bevorzugt im Bereich von 5 : 1 bis 1 : 5, ganz besonders bevorzugt im Bereich von 3 : 1 bis 1 : 3.

In einer weiteren bevorzugten Ausführungsform der Erfindung werden die Verbindungen gemäß Formel (1) als Lochtransportmaterial bzw. als Lochinjektionsmaterial eingesetzt. Die Verbindung wird dann bevorzugt in einer Lochtransport- bzw. in einer Lochinjektionsschicht in einer fluoreszierenden oder phosphoreszierenden OLED eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen einer Lochinjektionsschicht und einer Emissionsschicht liegt. Wenn die Verbindungen gemäß Formel (1) als Lochtransport- bzw. als Lochinjektionsmaterial verwendet werden, kann es bevorzugt sein, wenn sie mit Elektronenakzeptor-Verbindungen dotiert werden, beispielsweise mit F₄-TCNQ oder mit Verbindungen, wie in EP 1476881 oder EP 1596445 beschrieben.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig.

Die erfindungsgemäßen Verbindungen weisen bei Verwendung in organischen Elektrolumineszenzvorrichtungen folgende überraschende Vorteile gegenüber dem Stand der Technik auf:
1. Die Verbindungen weisen eine deutlich höhere Glasübergangstemperatur als CBP auf, welches gemäß dem Stand der Technik als Triplett-Matrixmaterial verwendet wird.
2. Auch die Lebensdauer der Vorrichtungen verbessert sich bei Verwendung der erfindungsgemäßen Verbindungen als Triplett-Matrixmaterialien.
3. Weiterhin verbessert sich die Effizienz der Vorrichtungen bei Verwendung der erfindungsgemäßen Verbindungen als Triplett-Matrixmaterialien.

Diese oben genannten Vorteile gehen nicht mit einer Verschlechterung der weiteren elektronischen Eigenschaften einher. Insbesondere zeigen die erfindungsgemäßen Vorrichtungen die gleiche Emissionsfarbe wie die Vorrichtungen gemäß dem Stand der Technik.

Im vorliegenden Anmeldetext wird auf die Verwendung der erfindungsgemäßen Verbindungen in Bezug auf OLEDs und PLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch für weitere Verwendungen in anderen elektronischen Vorrichtungen einzusetzen, z. B. für organische Feld-Effekt-Transistoren (O-FETs), organische Dünnfilmtransistoren (O-TFTs), organische lichtemittierende Transistoren (O-LETs), organische integrierte Schaltungen (O-ICs), organische Solarzellen (O-SCs), organische Feld-Quench-Devices (O-FQDs), lichtemittierende elektrochemische Zellen (LECs), organische Laserdioden (O-Laser) oder organische Photorezeptoren.

Die Verwendung der erfindungsgemäßen Verbindungen in den entsprechenden Vorrichtungen ebenso wie diese Vorrichtungen selbst sind ebenfalls ein Gegenstand der vorliegenden Erfindung.

Die Erfindung wird durch die nachfolgenden Beispiele näher erläutert, ohne sie dadurch einschränken zu wollen.

### Beispiele:

Die nachfolgenden Synthesen werden, sofern nicht anders angegeben, unter einer Schutzgasatmosphäre in getrockneten Lösungsmitteln durchgeführt. Die Edukte können von ALDRICH bezogen werden. 4-Brom-2-nitrobiphenyl und 2'-Nitro-p-terphenyl werden nach Literaturmethode hergestellt (M. Tavasli et al., Synthesis 2005, 1619-1624).

### Beispiel 1: Allgemeine Synthesevorschrift zur Carbazolsynthese

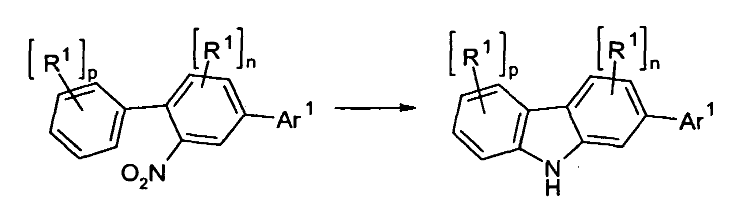

Eine Mischung aus 238 mmol des entsprechenden Nitroaromaten und 290.3 ml (1669 mmol) Triethylphosphit wird 12 h unter Rückfluss erhitzt. Anschließend wird das restliche Triethylphosphit abdestilliert (72-76 °C / 9 mm Hg). Der Rückstand wird mit Wasser/MeOH (1:1) versetzt, der Feststoff abfiltriert und umkristallisiert.

### Beispiel 2: Allgemeine Synthesevorschrift zur Hartwrig-Buchwald-Kupplung

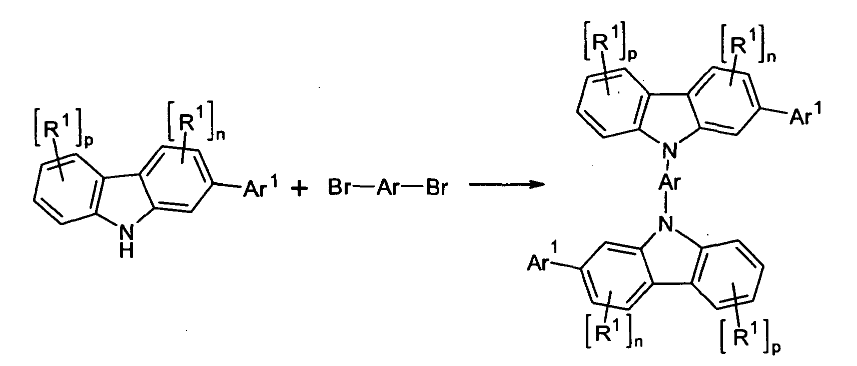

Eine entgaste Lösung von 176 mmol des Carbazol-Derivats und 64.2 mmol des Dibromaromaten in 250 ml Xylol wird 1 h lang mit N₂ gesättigt. Danach wird die Lösung zuerst mit 3 ml (12.2 mmol) P(^{t}Bu)₃, dann mit 0.5 g (2.45 mmol) Palladiumacetat versetzt und anschließend 81.9 g (956 mmol) K₃PO₄ im festen Zustand zugegeben. Die Reaktionsmischung wird 18 h unter Rückfluss erhitzt. Nach Abkühlen auf Raumtemperatur werden vorsichtig 1000 ml Wasser zugesetzt. Die organische Phase wird mit 4 x 50 mL H₂O gewaschen, über MgSO₄ getrocknet und die Lösungsmittel in Vakuum entfernt. Das reine Produkt erhält man durch Umkristallisation.

### Beispiel 3: Synthese von Bis-[2-phenylcarbazolyl]biphenyl (C1)

### a) Synthese von 2-Phenyl-9H-carbazol

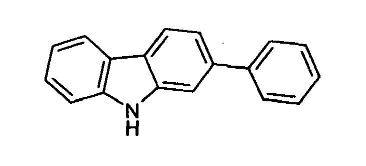

Die Synthese dieser Verbindung ist in der Literatur (M. Tavasli et al., Synthesis 2005, 1619-1624) beschrieben.

### b) Umsetzung mit 4,4'-Dibrombiphenyl zu Bis-[2-phenylcarbazolyl]biphenyl

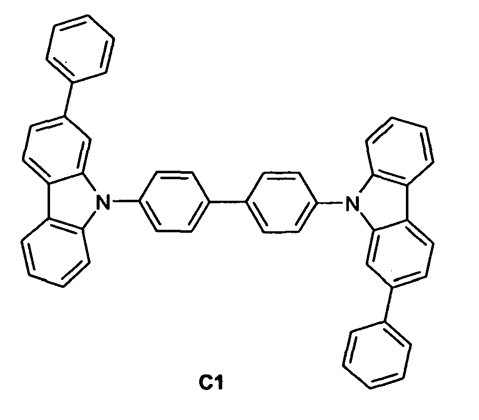

Die Synthese wird nach der allgemeinen Synthesevorschrift nach Beispiel 2 unter Verwendung von 4,4'-Dibrombiphenyl durchgeführt. Der erhaltene Feststoff wird mit Dioxan, dann mit MeOH und anschließend mit Essigsäureethylester heiß ausgerührt; Ausbeute: 39 g, 96 % d. Th.; Reinheit: 99.9 % nach HPLC.

### Beispiel 4: Synthese von 1,3-Bis-[2-phenylcarbazolyl]benzol (C2) durch Umsetzung von 2-Phenyl-9H-carbazol mit 1,3-Dibrombenzol

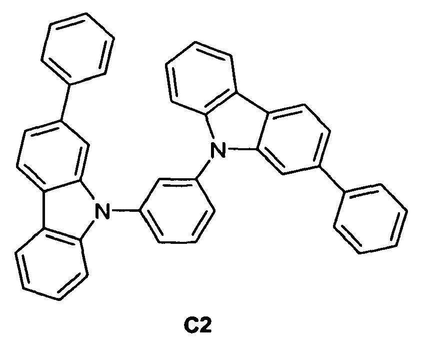

Die Synthese wird nach der allgemeinen Synthesevorschrift nach Beispiel 2 unter Verwendung von 1,3-Dibrombenzol durchgeführt. Der erhaltene Feststoff wird aus Hexan/CH₂Cl₂ (5:1) umkristallisiert. Die ausgefallenen Kristalle werden abgesaugt, mit etwas MeOH gewaschen und im Vakuum getrocknet; Ausbeute: 29.5 g, 91 % d. Th.; Reinheit: 99.9 % nach HPLC.

### Beispiel 5: Synthese von Bis-[2-o-tolylcarbazolyl]biphenyl (C3)

### a) Synthese von 2-Methyl-2'-nitro-p-terphenyl

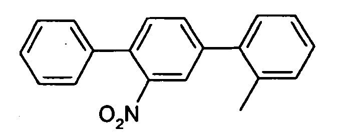

Eine gut gerührte, entgaste Suspension aus 25 g (183.8 mmol) o-Tolylboronsäure, 51.1 g (183.8 mmol) 4-Brom-2-nitrobiphenyl und 66.5 g (212.7 mmol) Kaliumcarbonat in einem Gemisch aus 250 ml Wasser und 250 ml THF wird mit 1.7 g (1.49 mmol) Pd(PPh₃)₄ versetzt und 17 h unter Rückfluss erhitzt. Nach Erkalten wird die organische Phase abgetrennt, dreimal mit 200 ml Wasser und einmal mit 200 ml gesättigter, wässriger Kochsalzlösung gewaschen, über Magnesiumsulfat getrocknet und zur Trockene einrotiert. Der graue Rückstand wird aus Hexan umkristallisiert. Die ausgefallenen Kristalle werden abgesaugt, mit etwas MeOH gewaschen und im Vakuum getrocknet; Ausbeute: 50.5 g, 95 % d. Th.; Reinheit: 99.5 % nach HPLC.

### b) Synthese von 2-o-Tolyl-9H-carbazol

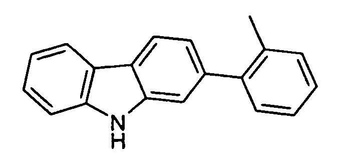

Die Synthese wird nach der allgemeinen Carbazolsynthese-Vorschrift nach Beispiel 1 durchgeführt, wobei das Terphenylderivat aus Beispiel 5a) verwendet wird. Der erhaltene Feststoff wird aus Hexan umkristallisiert. Die ausgefallenen Kristalle werden abgesaugt, mit etwas MeOH gewaschen und im Vakuum getrocknet; Ausbeute: 85 g, 80 % d. Th.; Reinheit: 98.0 % nach HPLC.

### c) Umsetzung mit 4,4'-Dibrombiphenyl zu Bis-[2-o-tolylcarbazolyl]biphenyl

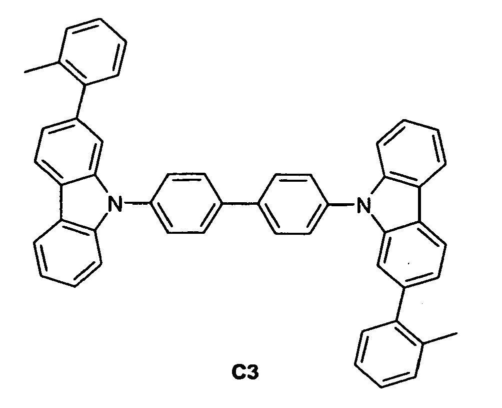

Die Synthese wird nach der allgemeinen Synthesevorschrift nach Beispiel 2 unter Verwendung von 4,4'-Dibrombiphenyl durchgeführt. Der erhaltene Feststoff wird aus Hexan/CH₂Cl₂ (5:1) umkristallisiert. Die ausgefallenen Kristalle werden abgesaugt, mit etwas MeOH gewaschen und im Vakuum getrocknet; Ausbeute: 44 g, 94 % d. Th.; Reinheit: 99.9 % nach HPLC.

### Beispiel 6: Synthese von Bis-[5-methyl-2-o-tolyl-carbazolyl]biphenyl (C4)

### a) Synthese von 2,2"-Dimethyl-2'-nitro-p-terphenyl

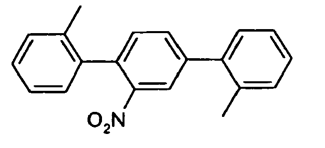

Eine gut gerührte, entgaste Suspension aus 155 g (1140 mmol) o-Tolylboronsäure, 133.4 g (474.9 mmol) 2,5-Dibrom-nitrobenzol und 305.3 g (1435 mmol) Kaliumcarbonat in einem Gemisch aus 250 ml Wasser und 250 ml THF wird mit 5.46 g (4.7 mmol) Pd(PPh₃)₄ versetzt und 20 h unter Rückfluss erhitzt. Nach Erkalten wird die organische Phase abgetrennt, dreimal mit 200 ml Wasser und einmal mit 200 ml gesättigter, wässriger Kochsalzlösung gewaschen, über Magnesiumsulfat getrocknet und im Vakuum zur Trockene einrotiert. Der graue Rückstand wird aus Hexan umkristallisiert. Die ausgefallenen Kristalle werden abgesaugt, mit etwas MeOH gewaschen und im Vakuum getrocknet; Ausbeute: 50.5 g, 97 % d. Th.; Reinheit: 99.2 % nach HPLC.

### b) Synthese von 5-Methyl-2-o-tolyl-9H-carbazol

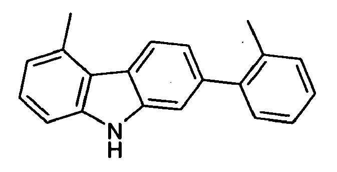

Die Synthese wird nach der allgemeinen Carbazolsynthese-Vorschrift nach Beispiel 1 durchgeführt, wobei das Terphenylderivat aus Beispiel 6a) verwendet wird. Der erhaltene Feststoff wird aus Hexan/CH₂Cl₂ (5:1) umkristallisiert. Die ausgefallenen Kristalle werden abgesaugt, mit etwas MeOH gewaschen und im Vakuum getrocknet; Ausbeute: 76 g, 70 % d. Th.; Reinheit: 97.0 % nach HPLC.

### c) Umsetzung mit 4,4'-Dibrombiphenyl zu Bis-[5-methyl-2-o-tolylcarbazolyl]biphenyl

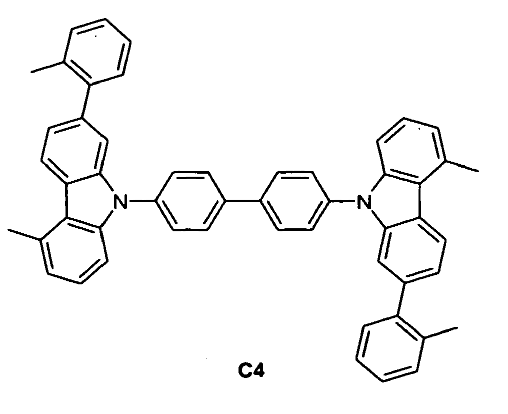

Die Synthese wird nach der allgemeinen Synthesevorschrift nach Beispiel 2 unter Verwendung von 4,4'-Dibrombiphenyl durchgeführt. Der erhaltene Feststoff wird aus Hexan/CH₂Cl₂ (5:1) umkristallisiert. Die ausgefallenen Kristalle werden abgesaugt, mit etwas MeOH gewaschen und im Vakuum getrocknet; Ausbeute: 44 g, 90 % d. Th.; Reinheit: 99.9 % nach HPLC.

### Beispiel 7: Synthese von Bis-[2-naphth-1-ylcarbazolyl]biphenyl (C5)

### a) Synthese von 4-Naphth-1-yl-2-nitro-biphenyl

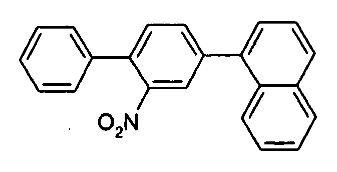

Eine gut gerührte, entgaste Suspension aus 46 g (268 mmol) 1-Naphthylboronsäure, 71 g (255.3 mmol) 4-Brom-2-nitrobiphenyl und 93 g (433.9 mmol) Kaliumcarbonat in einem Gemisch aus 700 ml Wasser und 700 ml THF wird mit 1.62 g (1.40 mmol) Pd(PPh₃)₄ versetzt und 17 h unter Rückfluss erhitzt. Nach Erkalten wird die organische Phase abgetrennt, dreimal mit 400 ml Wasser und einmal mit 400 ml gesättigter, wässriger Kochsalzlösung gewaschen, über Magnesiumsulfat getrocknet und im Vakuum zur Trockene einrotiert. Der graue Rückstand wird aus Hexan umkristallisiert. Die ausgefallenen Kristalle werden abgesaugt, mit etwas MeOH gewaschen und im Vakuum getrocknet; Ausbeute: 83.1 g, 97.9 % d. Th.; Reinheit: 99.0 % nach HPLC.

### b) Synthese von 2-Naphth-1-yl-9H-carbazol

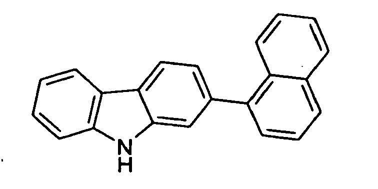

Die Synthese wird nach der allgemeinen Carbazolsynthese-Vorschrift nach Beispiel 1 durchgeführt, wobei die Verbindung aus Beispiel 7a) verwendet wird. Der erhaltene Feststoff wird aus Hexan/CH₂Cl₂ (5:1) umkristallisiert. Die ausgefallenen Kristalle werden abgesaugt, mit etwas MeOH gewaschen und im Vakuum getrocknet; Ausbeute: 55 g, 75 % d. Th.; Reinheit: 97.0 % nach HPLC.

### c) Umsetzung mit 4,4'-Dibrombiphenyl zu Bis-[2-naphth-1-yl-carbazolyl]biphenyl

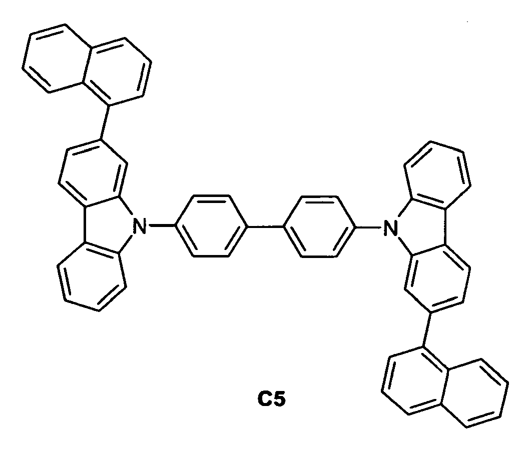

Die Synthese wird nach der allgemeinen Synthesevorschrift nach Beispiel 2 unter Verwendung von 4,4'-Dibrombiphenyl durchgeführt. Der erhaltene Feststoff wird aus Hexan/CH₂Cl₂ (5:1) umkristallisiert. Die ausgefallenen Kristalle werden abgesaugt, mit etwas MeOH gewaschen und im Vakuum getrocknet; Ausbeute: 41 g, 85 % d. Th.; Reinheit: 99.9 % nach HPLC.

### Beispiel 8: Synthese von Bis[9-naphth-1-yl-benzo[c]carbazolyl]biphenyl (C6)

### a) Synthese von 1-Nitro-2,5-dinaphth-1-yl -benzol

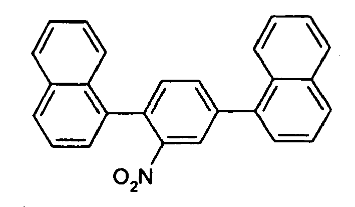

Eine gut gerührte, entgaste Suspension aus 67.8 g (190 mmol) 1-Naphthylboronsäure, 53.3 g (190 mmol) 2,5-Dibrom-nitrobenzol und 137.9 g (648.5 mmol) Kaliumcarbonat in einem Gemisch aus 250 ml Wasser und 250 ml THF wird mit 2.4 g (2.1 mmol) Pd(PPh₃)₄ versetzt und 20 h unter Rückfluss erhitzt. Nach Erkalten wird die organische Phase abgetrennt, dreimal mit 200 ml Wasser und einmal mit 200 ml gesättigter, wässriger Kochsalzlösung gewaschen, über Magnesiumsulfat getrocknet und im Vakuum zur Trockene einrotiert. Der graue Rückstand wird aus Hexan umkristallisiert. Die ausgefallenen Kristalle werden abgesaugt, mit etwas MeOH gewaschen und anschließend im Vakuum getrocknet; Ausbeute: 86.1 g, 71 % d. Th.; Reinheit: 98.4 % nach HPLC.

### b) Synthese von 9-Naphth-1-yl-7H-benzo[c]carbazol

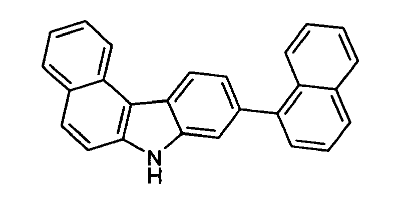

Die Synthese wird nach der allgemeinen Carbazolsynthese-Vorschrift nach Beispiel 1 durchgeführt, wobei die Verbindung aus Beispiel 8a) verwendet wird. Der erhaltene Feststoff wird aus Hexan/CH₂Cl₂ (5:1) umkristallisiert. Die ausgefallenen Kristalle werden abgesaugt, mit etwas MeOH gewaschen und anschließend im Vakuum getrocknet; Ausbeute: 49 g, 60 % d. Th.; Reinheit: 97.9 % nach HPLC.

### c) Umsetzung mit 4,4'-Dibrombiphenyl zu Bis[9-naphthyl-benzo[c]-carbazolyl]biphenyl

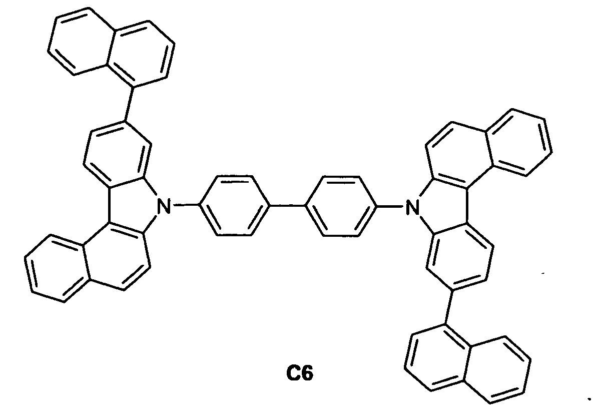

Die Synthese wird nach der allgemeinen Synthesevorschrift nach Beispiel 2 unter Verwendung von 4,4'-Dibrombiphenyl durchgeführt. Der erhaltene Feststoff wird Hexan/CH₂Cl₂ (5:1) umkristallisiert. Die ausgefallenen Kristalle werden abgesaugt, mit etwas MeOH gewaschen und im Vakuum getrocknet; Ausbeute: 49.5 g, 84 % d. Th.; Reinheit: 99.9 % nach HPLC.

### Beispiel 9: Bestimmung der Glasübergangstemperaturen

Von den Verbindungen **C1** bis **C6** und von CBP (Bis-4,4'-(N,N'-carbazolyl)-biphenyl) und 1,3-Bis(carbazolyl)benzol als Vergleichsverbindungen wird die Glasübergangstemperatur bestimmt. Die Bestimmung der Glasübergangstemperatur T_{g} erfolgt mit einem DSC-Gerät der Firma Netsch, DSC 204/1/G Phönix. Es werden dabei jeweils Proben in der Größe von 5-10 mg vermessen. Zur Bestimmung der Glasübergangstemperatur T_{g} wird die Probe nach dem Schmelzen aus dem DSC-Gerät entnommen und unverzüglich in flüssigen Stickstoff eingebracht, um eine maximale Kühlrate zu erreichen. Bei schnellem Heizen (20 K/min, bzw. wenn bei dieser Heizrate kein Ergebnis erhalten wird, bei 100 K/min) kann der T_{g} bestimmt werden. Die Ergebnisse sind in Tabelle 1 und Tabelle 2 zusammengefasst. Wie man sieht, sind die Glasübergangstemperaturen der erfindungsgemäßen Verbindungen deutlich höher als die der entsprechenden Vergleichsverbindungen, bei denen die Carbazolgruppen nicht durch Arylgruppen substituiert sind.

**Tabelle 1: Glasübergangstemperaturen**

| Verbindung | T_{g} in °C |
|---|---|
| CBP (Vergleich) | 112-116 |
| **C1** | 141 |
| **C3** | 132 |
| **C4** | |
| **C5** | 162 |
| **C6** | 198 |

**Tabelle 2: Glasübergangstemperaturen**

| Verbindung | T_{g} in °C |
|---|---|
| 1,3-Dicarbazolylbenzol (Vergleich) | 66 |
| **C2** | 110 |

### Beispiel 10: Herstellung und Charakterisierung von organischen Elektrolumineszenzvorrichtungen, die die erfindungsgemäßen Verbindungen enthalten

Erfindungsgemäße Elektrolumineszenzvorrichtungen können, wie beispielsweise in WO 05/003253 beschrieben, dargestellt werden. Hier werden die Ergebnisse verschiedener OLEDs gegenübergestellt. Der grundlegende Aufbau, die verwendeten Materialien, der Dotierungsgrad und ihre Schichtdicken sind zur besseren Vergleichbarkeit identisch. Es wird ausschließlich der Host in der Emissionsschicht variiert. Das erste Beispiel beschreibt einen Vergleichsstandard nach dem Stand der Technik, bei dem die Emissionsschicht aus dem Wirtsmaterial CBP und dem Gastmaterial (Dotanden) Ir(piq)₃ besteht. Des Weiteren wird eine OLED mit einer Emitterschicht bestehend aus dem Wirtsmaterial **C1, C2, C4** oder **C5** und dem Gastmaterialien (Dotanden) Ir(piq)₃ und beschrieben. Analog dem o. g. allgemeinen Verfahren, werden OLEDs mit folgendem Aufbau erzeugt:
- Lochinjektionsschicht (HIL): 10 nm 2,2',7,7'-Tetrakis(di-para-tolyl- amino)spiro-9,9'-bifluoren
- Lochtransportschicht (HTL): 30 nm NPB (N-Naphthyl-N-phenyl-4,4'- diaminobiphenyl)
- Emissionschicht (EML): Host: CPB (aufgedampft; von ALDRICH und weiter gereinigt, zweimal sublimiert; 4,4'-Bis-(N-carbazolyl)biphenyl) als Vergleich oder **C1, C2, C4** oder **C5.** Dotand: Ir(piq)₃ (10 % Dotierung, aufgedampft; synthetisiert nach WO 03/0068526); siehe Tabelle 3
- Lochblockierschicht (HBL): BAlq 10 nm (bezogen von ERay, Bis(2- methyl-8-chinolinolato)(para-phenyl- phenolato)aluminium(III)
- Elektronenleiter (ETL): 20 nm AlQ₃ (bezogen von ERay, Tris- (chinolinato)aluminium(III))
- Kathode: 1 nm LiF, darauf 150 nm Al.

Die Struktur von Ir(piq)₃ ist der Übersichtlichkeit halber im Folgenden abgebildet:

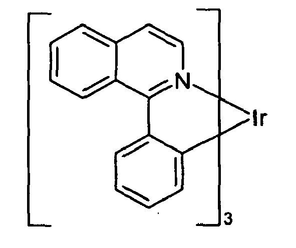

Diese noch nicht optimierten OLEDs werden standardmäßig charakterisiert; hierfür werden die Elektrolumineszenzspektren, die Effizienz (gemessen in cd/A) in Abhängigkeit von der Helligkeit, berechnet aus Strom-Spannungs-Helligkeit-Kennlinien (IUL-Kennlinien), und die Lebensdauer bestimmt.

Mit OLEDs hergestellt mit dem Standard-Host CBP erhält man unter den oben beschriebenen Bedingungen typischerweise eine maximale Effizienz von etwa 7.9 cd/A bei Farbkoordinaten von CIE: x = 0.68, y = 0.32. Für die Referenzleuchtdichte von 1000 cd/m² werden Spannungen von 6.0 V benötigt. Die Lebensdauer beträgt etwa 5000 h bei einer Anfangsleuchtdichte von 1000 cd/m² (s. Tabelle 3). Im Gegensatz dazu zeigen OLEDs hergestellt mit den erfindungsgemäßen Hostmaterialien **C1, C2, C4** und **C5** bei ansonsten gleichem Aufbau maximale Effizienzen von 8.3 cd/A bei Farbkoordinaten von CIE: x = 0.68, y = 0.32, wobei die benötigte Spannung für die Referenzleuchtdichte von 1000 cd/m² bei bis zu 5.0 V liegt (s. Tabelle 3). Die Lebensdauer bei einer Anfangsleuchtdichte von 1000 cd/m² ist mit bis zu 11000 h höher als mit dem Referenzmaterial CBP (s. Tabelle 3).

**Tabelle 3: Device-Ergebnisse mit erfindungsgemäßen Hostmaterialien mit Ir(piq)₃ als Dotand**

| Experiment | EML | Max. Effizienz [cd/A] | Spannung [V] bei 100 cd/m² | CIE (x, y) | Lebensdauer [h] Anfangshelligkeit 1000 [cd/m²] |
|---|---|---|---|---|---|
| Beispiel 11 (Vergleich) | CBP:10% Ir(piq)₃ (30 nm) | 7.9 | 6.0 | 0.68/0.32 | 5000 |
| Beispiel 12 | **C1**:10% Ir(piq)₃ (30 nm) | 8.3 | 5.9 | 0.68/0.32 | 11000 |
| Beispiel 13 | **C2**:10% Ir(piq)₃ (30 nm) | 7.5 | 5.6 | 0.68/0.32 | 7000 |
| Beispiel 14 | **C4**:10% Ir(piq)₃ (30 nm) | 8.2 | 5.2 | 0.68/0.32 | 5000 |
| Beispiel 15 | **C5**:10% Ir(piq)₃ (30 nm) | 8.1 | 5.0 | 0.68/0.32 | 9000 |

Analog zu den oben aufgeführten Beispielen 11 bis 15 wurden weitere organische Elektrolumineszenzvorrichtungen hergestellt, die den gleichen Deviceaufbau aufweisen wie die oben genannten Vorrichtungen, in denen jedoch als Emissionsmaterial (Dotand) Ir(ppy)₃ (Tris(phenylpyridin)iridium, synthetisiert nach WO 04/085449) verwendet wird.

Die Struktur von Ir(ppy)₃ ist der Übersichtlichkeit halber im Folgenden abgebildet:

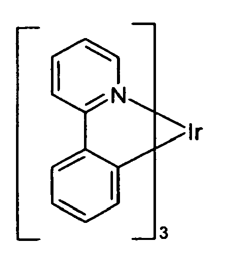

Mit OLEDs hergestellt mit dem Standard-Host CBP erhält man unter den oben beschriebenen Bedingungen typischerweise eine maximale Effizienz von etwa 25 cd/A bei Farbkoordinaten von CIE: x = 0.30, y = 0.60. Für die Referenzleuchtdichte von 1000 cd/m² werden Spannungen von 5.3 V benötigt. Die Lebensdauer beträgt etwa 2400 h bei einer Anfangsleuchtdichte von 1000 cd/m² (s. Tabelle 4). Im Gegensatz dazu zeigen OLEDs hergestellt mit dem erfindungsgemäßem Host **C1** maximale Effizienzen von 27 cd/A bei Farbkoordinaten von CIE: x = 0.30, y = 0.60, wobei die benötigte Spannung für die Referenzleuchtdichte von 1000 cd/m² bei 4.7 V liegt (s. Tabelle 4). Die Lebensdauer bei einer Anfangsleuchtdichte von 1000 cd/m² ist mit 3000 h höher als mit dem Referenzmaterial CBP (s. Tabelle 4).

**Tabelle 4: Device-Ergebnisse mit erfindungsgemäßen Hostmaterialien mit Ir(ppy)₃ als Dotand**

| Experiment | EML | Max. Effizienz [cd/A] | Spannung [V] bei 100 cd/m² | CIE (x, y) | Lebensdauer [h] Anfangshelligkeit 1000 [cd/m²] |
|---|---|---|---|---|---|
| Beispiel 16 (Vergleich) | CBP:5% Ir(ppy)₃ (30 nm) | 25 | 5.3 | 0.30/0.60 | 2400 |
| Beispiel 17 | **C1**:5% Ir(ppy)₃ (30 nm) | 27 | 4.7 | 0.30/0.60 | 3000 |

Analog zum oben aufgeführten Beispiel 17 wurde eine weitere organische Elektrolumineszenzvorrichtungen hergestellt, die den gleichen Deviceaufbau und das gleiche Emissionsmaterial Ir(ppy)₃ aufweist wie die oben genannten Vorrichtung, in der jedoch als Matrixmaterial (Wirtsmaterial) eine Mischung aus der erfindungsgemäßen Verbindung **C1** und Bis(9,9'-spiro-bifluoren-2-yl)keton (synthetisiert nach WO 04/093207) verwendet wird.

Die Struktur von Bis(9,9'-spiro-bifluoren-2-yl)keton ist der Übersichtlichkeit halber im Folgenden abgebildet:

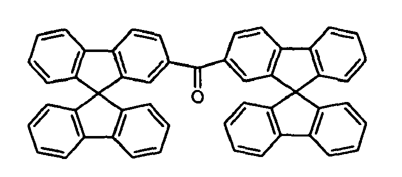

Mit OLEDs hergestellt mit einer Mischung aus dem erfindungsgemäßen Host **C1** und Bis(9,9'-spiro-bifluoren-2-yl)keton erhält man maximale Effizienzen von 37 cd/A bei Farbkoordinaten von CIE: x = 0.34, y = 0.60, wobei die benötigte Spannung für die Referenzleuchtdichte von 1000 cd/m² nur bei 3.2 V liegt (s. Tabelle 5). Die Lebensdauer bei einer Anfangsleuchtdichte von 1000 cd/m² beträgt 14000 h (s. Tabelle 5). Mit einer Mischung der Hostmaterialien ist also nochmals eine weitere Steigerung der Effizienz und der Lebensdauer möglich.

**Tabelle 5: Device-Ergebnisse mit erfindungsgemäßen Hostmaterialien mit Ir(ppy)₃ als Dotand**

| Experiment | EML | Max. Effizienz [cd/A] | Spannung [V] bei 100 cd/m² | CIE (x, y) | Lebensdauer [h] Anfangshelligkeit 1000 [cd/m²] |
|---|---|---|---|---|---|
| Beispiel 18 | 47.5% **C1**, 47.5% Spiroketon 5% Ir(ppy)₃ (30 nm) | 37 | 3.2 | 0.34/0.60 | 14000 |

## Patentansprüche

1. Verbindungen gemäß Formel (1), 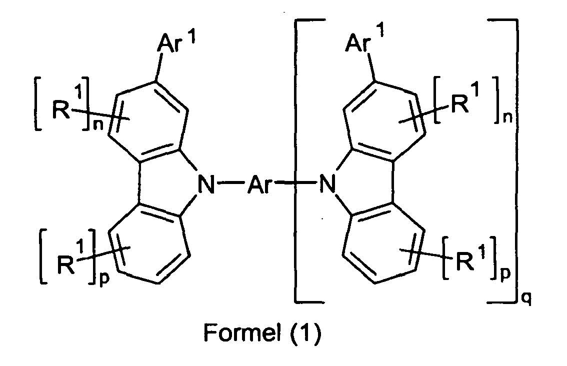 wobei für die verwendeten Symbole und Indizes gilt:
Ar ist bei jedem Auftreten ein aromatisches oder hetero- aromatisches Ringsystem mit 5 bis 60 aromatischen Ring- atomen, welches mit einem oder mehreren Resten R¹ substituiert sein kann;
Ar¹ ist bei jedem Auftreten gleich oder verschieden ein aromatisches oder heteroaromatisches Ringsystem mit 5 bis 60 aromatischen Ringatomen, welches mit einem oder mehreren Resten R substituiert sein kann;
R ist bei jedem Auftreten gleich oder verschieden Cl, Br, I, N(Ar²)₂, CN, NO₂, Si(R²)₃, B(OR²)₂, C(=O)Ar², P(=O)(Ar²)₂, S(=O)Ar², S(=O)₂Ar², -CR²=CR²(Ar²), OSO₂R², eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C=C , Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aro- matischen Ringatomen, die durch einen oder mehrere Reste R² substituiert sein kann, oder eine Kombination dieser Systeme; dabei können zwei oder mehrere Substituenten R auch miteinander ein mono- oder polycyclisches aliphatisches oder aromatisches Ringsystem bilden;
R¹ ist bei jedem Auftreten gleich oder verschieden R, eine Gruppe Ar¹ oder F;
Ar² ist bei jedem Auftreten gleich oder verschieden ein aromatisches oder heteroaromatisches Ringsystem mit 5 bis 60 aromatischen Ringatomen, welches mit einem oder mehreren Resten R² substituiert sein kann;
R² ist bei jedem Auftreten gleich oder verschieden H oder ein aliphatischer, aromatischer und/oder heteroaromatischer Kohlenwasserstoffrest mit 1 bis 20 C-Atomen; dabei können zwei oder mehrere Substituenten R² auch miteinander ein mono- oder polycyclisches aliphatisches oder aromatisches Ringsystem bilden;
n ist bei jedem Auftreten gleich oder verschieden 0, 1, 2 oder 3;
p ist bei jedem Auftreten gleich oder verschieden 0, 1, 2, 3 oder 4;
q ist 1, 2, 3, 4 oder 5.

2. Verbindungen nach Anspruch 1, **dadurch gekennzeichnet, dass** die Indizes n, gleich oder verschieden bei jedem Auftreten, 0 oder 1 sind, bevorzugt 0.

3. Verbindungen nach Anspruch 1 oder 2, ausgewählt aus den Strukturen der Formeln (2) bis (7), 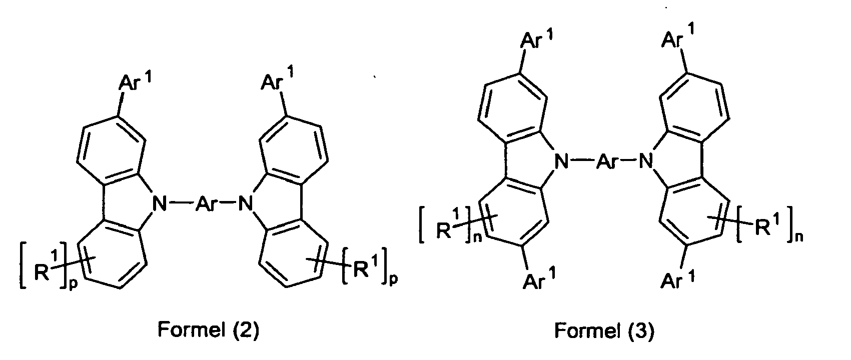 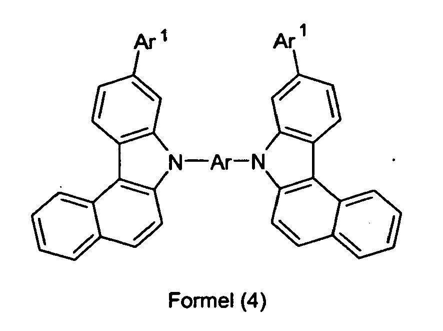 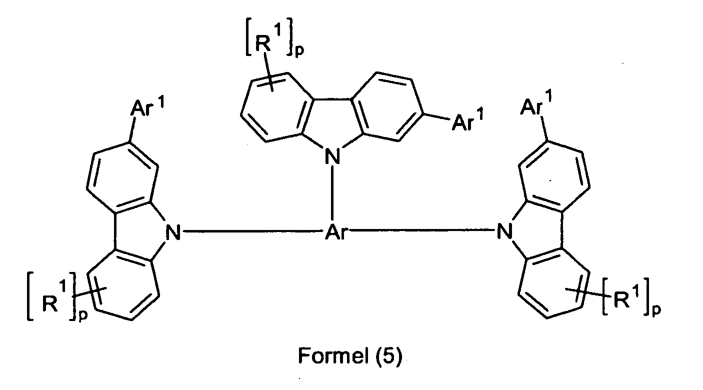 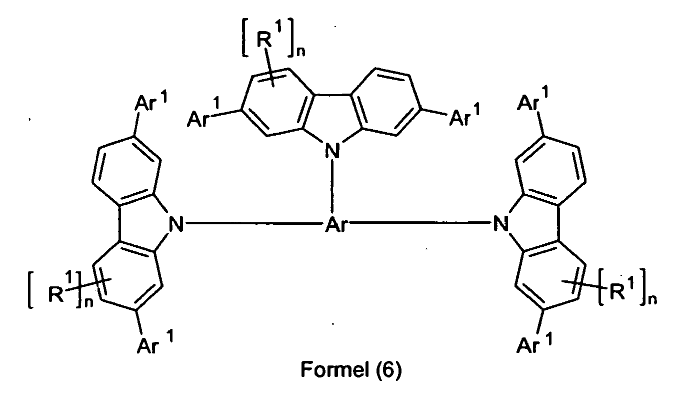 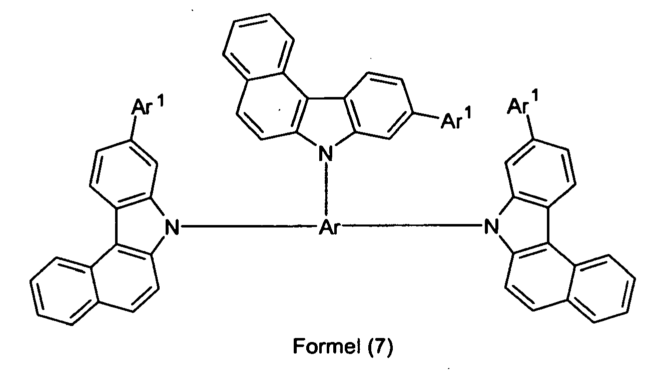 wobei die Symbole und Indizes die in Anspruch 1 aufgeführten Bedeutungen haben.

4. Verbindungen nach einem oder mehreren der Ansprüche 1 bis 3, **dadurch gekennzeichnet, dass** der Index p gleich oder verschieden bei jedem Auftreten 0, 1 oder 2, bevorzugt 0 oder 1 ist.

5. Verbindungen nach einem oder mehreren der Ansprüche 1 bis 4, **dadurch gekennzeichnet, dass** der Substituent R¹ in der 5-Position oder in der 7-Position des Carbazols gebunden ist, wenn der Index p = 1 ist, bzw. dass die Substituenten R¹ in der 5- und in der 7-Position des Carbazols gebunden sind, wenn der Index p = 2 ist.

6. Verbindungen nach einem oder mehreren der Ansprüche 1 bis 5, **dadurch gekennzeichnet, dass** die Gruppen Ar und Ar¹ aromatische Ringsysteme sind, welche aufgebaut sind aus Phenyl- und/oder Naphthylgruppen oder Verknüpfungen dieser Systeme, oder heteroaromatische Gruppen mit nicht mehr als zwei kondensierten aromatischen bzw. heteroaromatischen Ringen oder Verknüpfungen dieser Systeme oder Carbazol.

7. Verbindungen nach einem oder mehreren der Ansprüche 1 bis 6, **dadurch gekennzeichnet, dass** die Gruppe Ar gewählt ist aus 1,2-Phenylen, 1,3-Phenylen, 1,4-Phenylen, 1,3,5-Benzol, 3,3'-Biphenyl, 4,4'-Biphenyl, 1,3,5-Triphenylbenzol, Triphenylamin, 2,7-Fluorenylen, welches durch einen oder mehrere Reste R¹ substituiert sein kann, 2,7-Spirobifluorenylen, welches durch einen oder mehrere Reste R¹ substituiert sein kann, Indenofluorenylen, welches durch einen oder mehrere Reste R¹ substituiert sein kann, 4,4"'-(1,1':2',1",2",1"'-Quarterphenyl), 4,4'-(2,2'-Dimethylbiphenyl), 4,4'-(1,1'-Binaphthyl), 4,4'-Stilbenyl oder Dihydrophenanthrenyl, welches durch einen oder mehrere Reste R¹ substituiert sein kann.

8. Verbindungen nach einem oder mehreren der Ansprüche 1 bis 7, **dadurch gekennzeichnet, dass** die Gruppen Ar¹ gleich oder verschieden gewählt sind aus Phenyl, 1-Naphthyl, 2-Naphthyl, Triphenylamin, 2-Carbazolyl, 3-Carbazolyl, 9-Carbazolyl, Naphthyldiphenylamin oder Dinaphthylphenylamin, welche jeweils durch einen oder mehrere Reste R substituiert sein können.

9. Verbindungen nach einem oder mehreren der Ansprüche 1 bis 8, **dadurch gekennzeichnet, dass** das Symbol R gleich oder verschieden bei jedem Auftreten für H, N(Ar²)₂, eine geradkettige Alkylgruppe mit 1 bis 5 C-Atomen oder verzweigte Alkylgruppe mit 3 bis 5 C-Atomen, wobei jeweils eine oder mehrere nicht benachbarte CH₂-Gruppen durch -R²C=CR²- oder -O- ersetzt sein können und wobei ein oder mehrere H-Atome durch F ersetzt sein können, oder eine Arylgruppe mit 6 bis 16 C-Atomen oder Heteroarylgruppe mit 2 bis 16 C-Atomen oder eine Spirobifluorengruppe, die jeweils mit einem oder mehreren Resten R² substituiert sein kann, oder eine Kombination aus zwei dieser Systeme steht.

10. Verbindungen nach einem oder mehreren der Ansprüche 1 bis 9, **dadurch gekennzeichnet, dass** es sich um symmetrische Verbindungen handelt, in denen alle Symbole Ar¹ gleich sind.

11. Verfahren zur Herstellung von Verbindungen nach einem oder mehreren der Ansprüche 1 bis 10 durch Umsetzung eines 4-Aryl-2-nitro-1,1'-biphenyl oder 4-Heteroaryl-2-nitro-1,1'-biphenyl, wobei die Aryl- bzw. Heteroarylgruppe durch einen oder mehrere Reste R substituiert sein kann und das Biphenyl durch einen oder mehrere Reste R¹ substituiert sein kann, mit einem Trialkylphosphit, wobei die Alkylgruppen gleich oder verschieden bei jedem Auftreten 1 bis 10 C-Atome aufweisen, zum entsprechenden Carbazol, gefolgt von einer Hartwig-Buchwald-Kupplung mit einer aromatischen Verbindung Ar, welche mindestens zwei reaktive Gruppen aufweist, welche bevorzugt gewählt sind aus Chlor, Brom, Iod, Triflat, Tosylat oder OSO₂-R², wobei R² dieselbe Bedeutung hat, wie in Anspruch 1 ausgeführt.

12. Verwendung von Verbindungen nach einem oder mehreren der Ansprüche 1 bis 10 in organischen elektronischen Vorrichtungen, insbesondere in organischen Elektrolumineszenzvorrichtungen.

13. Organische elektronische Vorrichtung, insbesondere organische Elektrolumineszenzvorrichtung (OLED, PLED), organischer Feld-Effekt-Transistor (O-FET), organischer Dünnfilmtransistor (O-TFT), organischer lichtemittierender Transistor (O-LET), organische integrierte Schaltung (O-IC), organische Solarzelle (O-SC), organisches Feld-Quench-Device (O-FQD), lichtemittierende elektrochemische Zelle (LEC), organische Laserdiode (O-Laser) oder organischer Photorezeptor, enthaltend in mindestens einer Schicht mindestens eine Verbindung nach einem oder mehreren der Ansprüche 1 bis 10.

14. Organische Elektrolumineszenzvorrichtung nach Anspruch 13, enthaltend Kathode, Anode und mindestens eine emittierende Schicht und gegebenenfalls weitere Schichten, gewählt aus jeweils einer oder mehreren Lochinjektionsschichten, Lochtransportschichten, Lochblockierschichten, Elektronentransportschichten, Elektroneninjektionsschichten und/oder Ladungserzeugungsschichten (Charge-Generation Layers).

15. Organische Elektrolumineszenzvorrichtung nach Anspruch 14, **dadurch gekennzeichnet, dass** die Verbindung nach einem oder mehreren der Ansprüche 1 bis 10 als Matrix für phosphoreszierende Dotanden eingesetzt wird.

16. Organische Elektrolumineszenzvorrichtung nach Anspuch 15, **dadurch gekennzeichnet, dass** dass die Verbindung nach einem oder mehreren der Ansprüche 1 bis 10 zusammen mit einem aromatischen Keton, einem aromatischen Phosphinoxid, einem aromatischen Sulfoxid oder einem aromatischen Sulfon als Matrix für phosphorezierende Dotanden eingesetzt wird.

17. Organische Elektrolumineszenzvorrichtung nach Anspruch 15 oder 16, **dadurch gekennzeichnet, dass** der phosphoreszierende Dotand mindestens eine Verbindung enthält, die bei geeigneter Anregung Licht emittiert und mindestens ein Atom der Ordnungszahl größer 20, bevorzugt größer 38 und kleiner 84 enthält.

18. Organische Elektrolumineszenzvorrichtung nach einem oder mehreren der Ansprüche 14 bis 17, **dadurch gekennzeichnet, dass** die Verbindung nach einem oder mehreren der Ansprüche 1 bis 10 als Lochtransportmaterial bzw. als Lochinjektionsmaterial eingesetzt wird, bevorzugt in einer Lochtransportschicht bzw. in einer Lochinjektionsschicht.

## Claims

1. Compounds of the formula (1) 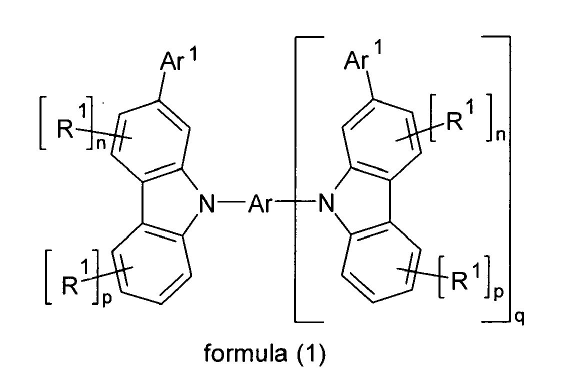 where the following applies to the symbols and indices used:
Ar is on each occurrence an aromatic or heteroaromatic ring system having 5 to 60 aromatic ring atoms, which may be substituted by one or more radicals R¹;
Ar¹ is on each occurrence, identically or differently, an aromatic or heteroaromatic ring system having 5 to 60 aromatic ring atoms, which may be substituted by one or more radicals R;
R is on each occurrence, identically or differently, Cl, Br, I, N(Ar²)₂, CN, NO₂, Si(R²)_{3,} B(OR²)₂, C(=O)Ar², P(=O)(Ar²)₂, S(=O)Ar², S(=O)₂Ar², -CR²=CR²(Ar²), OSO₂R², a straight- chain alkyl, alkoxy or thioalkoxy group having 1 to 40 C atoms or a branched or cyclic alkyl, alkoxy or thioalkoxy group hav- ing 3 to 40 C atoms, each of which may be substituted by one or more radicals R², where one or more non-adjacent CH₂ groups may be replaced by R²C=CR², C=C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R² or a combination of these systems; two or more substituents R here may also form a mono- or poly- cyclic aliphatic or aromatic ring system with one another;
R¹ is on each occurrence, identically or differently, R, a group Ar¹ or F;
Ar² is on each occurrence, identically or differently, an aromatic or heteroaromatic ring system having 5 to 60 aromatic ring atoms, which may be substituted by one or more radicals R²;
R² is on each occurrence, identically or differently, H or an ali- phatic, aromatic and/or heteroaromatic hydrocarbon radical having 1 to 20 C atoms; two or more substituents R² here may also form a mono- or polycyclic aliphatic or aromatic ring system with one another;
n is on each occurrence, identically or differently, 0, 1, 2 or 3;
p is on each occurrence, identically or differently, 0, 1, 2, 3 or 4;
q is 1, 2, 3, 4 or 5.

2. Compounds according to Claim 1, **characterised in that** the indices n, identically or differently on each occurrence, are 0 or 1, preferably 0.

3. Compounds according to Claim 1 or 2, selected from the structures of the formulae (2) to (7), 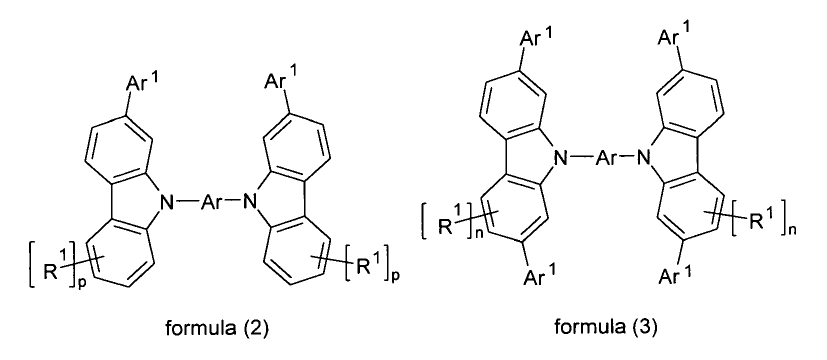 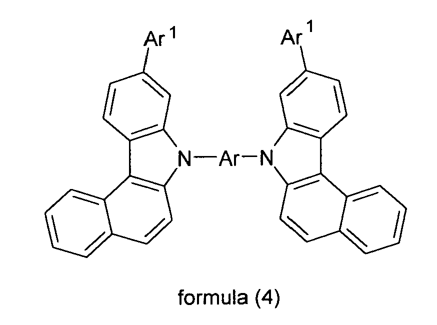 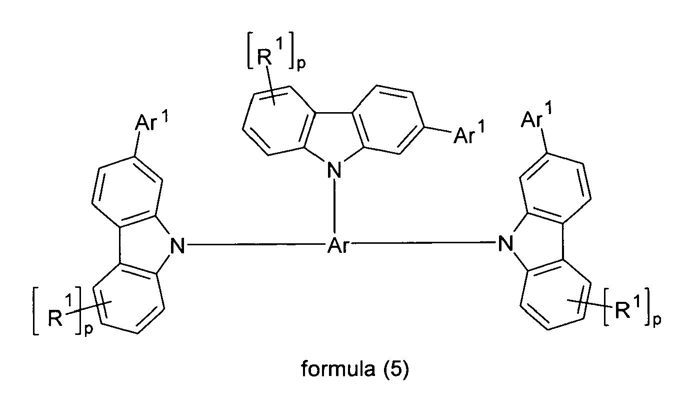 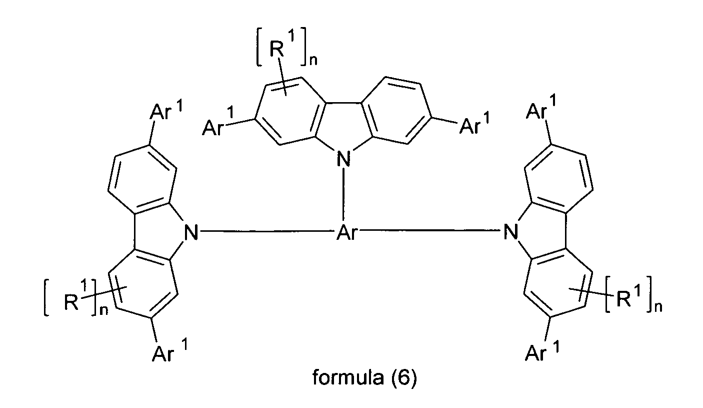 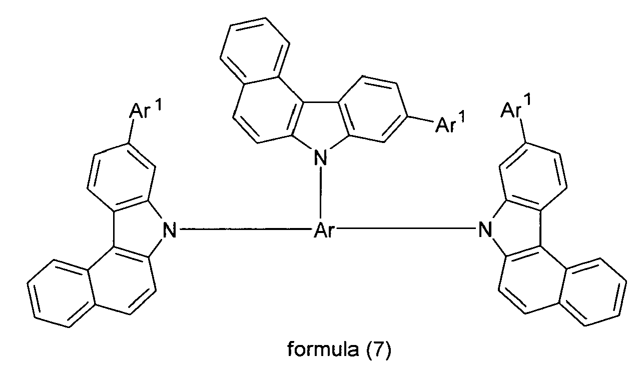 where the symbols and indices have the meanings indicated in Claim 1.

4. Compounds according to one or more of Claims 1 to 3, **characterised in that** the index p, identically or differently on each occurrence, is 0, 1 or 2, preferably 0 or 1.

5. Compounds according to one or more of Claims 1 to 4, **characterised in that** the substituent R¹ is bonded in the 5-position or 7-position of the carbazole if the index p = 1, or **in that** the substituents R¹ are bonded in the 5- and 7-position of the carbazole if the index p = 2.

6. Compounds according to one or more of Claims 1 to 5, **characterised in that** the groups Ar and Ar¹ are aromatic ring systems built up from phenyl and/or naphthyl groups or linked systems of this type, or heteroaromatic groups having not more than two condensed aromatic or heteroaromatic rings or linked systems of this type or carbazole.

7. Compounds according to one or more of Claims 1 to 6, **characterised in that** the group Ar is selected from 1,2-phenylene, 1,3-phenylene, 1,4-phenylene, 1,3,5-benzene, 3,3'-biphenyl, 4,4'-biphenyl, 1,3,5-triphenylbenzene, triphenylamine, 2,7-fluorenylene, each of which may be substituted by one or more radicals R¹, 2,7-spirobifluorenylene, which may be substituted by one or more radicals R¹, indenofluorenylene, which may be substituted by one or more radicals R¹, 4,4'"-(1,1': 2', 1 ",2", 1'"-quaterphenyl), 4,4'-(2,2'-dimethylbiphenyl), 4,4'-(1,1'-binaphthyl), 4,4'-stilbenyl or dihydrophenanthrenyl, each of which may be substituted by one or more radicals R¹.

8. Compounds according to one or more of Claims 1 to 7, **characterised in that** the groups Ar¹ are selected, identically or differently, from phenyl, 1-naphthyl, 2-naphthyl, triphenylamine, 2-carbazolyl, 3-carbazolyl, 9-carbazolyl, naphthyldiphenylamine or dinaphthylphenylamine, each of which may be substituted by one or more radicals R.

9. Compounds according to one or more of Claims 1 to 8, **characterised in that** the symbol R stands, identically or differently on each occurrence, for H, N(Ar²)₂, a straight-chain alkyl group having 1 to 5 C atoms or a branched alkyl group having 3 to 5 C atoms, where in each case one or more non-adjacent CH₂ groups may be replaced by -R²C=CR² - or -O- and where one or more H atoms may be replaced by F, or an aryl group having 6 to 16 C atoms or a heteroaryl group having 2 to 16 C atoms or a spirobifluorene group, each of which may be substituted by one or more radicals R², or a combination of two of these systems.

10. Compounds according to one or more of Claims 1 to 9, **characterised in that** they are symmetrical compounds in which all symbols Ar¹ are identical.

11. Process for the preparation of compounds according to one or more of Claims 1 to 10 by reaction of a 4-aryl-2-nitro-1,1'-biphenyl or 4-heteroaryl-2-nitro-1,1'-biphenyl, where the aryl or heteroaryl group may be substituted by one or more radicals R and the biphenyl may be substituted by one or more radicals R¹, with a trialkyl phosphite, where the alkyl groups, identically or differently on each occurrence, have 1 to 10 C atoms, to give the corresponding carbazole, followed by a Hartwig-Buchwald coupling to an aromatic compound Ar which has at least two reactive groups, which are preferably selected from chlorine, bromine, iodine, triflate, tosylate or OSO₂-R², where R² has the same meaning as indicated in Claim 1.

12. Use of compounds according to one or more of Claims 1 to 10 in organic electronic devices, in particular in organic electroluminescent devices.

13. Organic electronic device, in particular organic electroluminescent device (OLED, PLED), organic field-effect transistor (O-FET), organic thin-film transistor (O-TFT), organic light-emitting transistor (O-LET), organic integrated circuit (O-IC), organic solar cell (O-SC), organic field-quench device (O-FQD), light-emitting electrochemical cell (LEC), organic laser diode (O-laser) or organic photoreceptor, comprising at least one compound according to one or more of Claims 1 to 10 in at least one layer.

14. Organic electroluminescent device according to Claim 13, comprising cathode, anode and at least one emitting layer and optionally further layers, in each case selected from one or more hole-injection layers, hole-transport layers, hole-blocking layers, electron-transport layers, electron-injection layers and/or charge-generation layers.

15. Organic electroluminescent device according to Claim 14, **characterised in that** the compound according to one or more of Claims 1 to 10 is employed as matrix for phosphorescent dopants.

16. Organic electroluminescent device according to Claim 15, **characterised in that** the compound according to one or more of Claims 1 to 10 is employed together with an aromatic ketone, an aromatic phosphine oxide, an aromatic sulfoxide or an aromatic sulfone as matrix for phosphorescent dopants.

17. Organic electroluminescent device according to Claim 15 or 16, **characterised in that** the phosphorescent dopant comprises at least one compound which emits light on suitable excitation and contains at least one atom having an atomic number of greater than 20, preferably greater than 38 and less than 84.

18. Organic electroluminescent device according to one or more of Claims 14 to 17, **characterised in that** the compound according to one or more of Claims 1 to 10 is employed as hole-transport material or as hole-injection material, preferably in a hole-transport layer or in a hole-injection layer.

## Revendications

1. Composés de la formule (1) 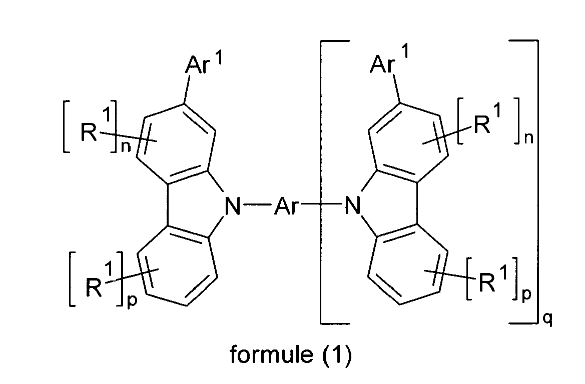 dans laquelle ce qui suit s'applique aux symboles et indices utilisés :
Ar est à chaque occurrence un système de cycle aromatique ou hétéroaromatique ayant 5 à 60 atomes de cycle aromatique, qui peuvent être substitués par un ou plusieurs radicaux R¹;
Ar¹ est à chaque occurrence, de façon identique ou différente, un système de cycle aromatique ou hétéroaromatique ayant 5 à 60 atomes de cycle aromatique, qui peuvent être substitués par un ou plusieurs radicaux R;
R est à chaque occurrence, de façon identique ou différente, CI, Br, I, N(Ar²)₂, CN, NO₂, Si(R²)₃, B(OR²)₂, C(=O)Ar², P(=O)(Ar²)₂, S(=O)Ar², S(=O)₂Ar², -CR²=CR²(Ar²), OSO₂R², un groupe alkyle, alcoxy ou thioalcoxy en chaîne droite ayant 1 à 40 atomes de C ou un groupe alkyle, alcoxy ou thioalcoxy ramifié ou cyclique ayant 3 à 40 atomes de C, chacun de ceux-ci peut être substitué par un ou plusieurs radicaux R², dans lequel un ou plusieurs groupes CH₂ non adjacents peu- vent être remplacés par R²C=CR², C≡C, Si(R²)₂, Ge(R²)₂, Sn(R²)₂, C=O, C=S, C=Se, C=NR², P(=O)(R²), SO, SO₂, NR², O, S ou CONR² et dans lequel un ou plusieurs atomes de H peuvent être remplacés par F, Cl, Br, I, CN ou NO₂, ou un système de cycle aromatique ou hétéroaromatique ayant 5 à 60 atomes de cycles aromatique, qui peuvent dans chaque cas être substitués par un ou plusieurs radicaux R², ou un groupe aryloxy ou hétéroaryloxy ayant 5 à 60 atomes de cycle aromatique, qui peuvent être substitués par un ou plu- sieurs radicaux R², ou une combinaison de ces systèmes ; deux ou plus de deux substituants R peuvent ici également former un système de cycle aliphatique ou aromatique mono- ou polycyclique l'un avec l'autre ;
R¹ est à chaque occurrence, de façon identique ou différente, R, un groupe Ar¹ ou F;
Ar² est à chaque occurrence, de façon identique ou différente, un système de cycle aromatique ou hétéroaromatique ayant 5 à 60 atomes de cycle aromatique, qui peuvent être substitués par un ou plusieurs radicaux R²;
R² est à chaque occurrence, de façon identique ou différente, H ou un radical hydrocarbure aliphatique, aromatique et/ou hétéroaromatique ayant 1 à 20 atomes de C ; deux ou plus de deux substituants R² peuvent ici également former un système de cycle aliphatique ou aromatique mono- ou poly- cyclique l'un avec l'autre ;
n est à chaque occurrence, de façon identique ou différente, 0, 1, 2 ou 3;
p est à chaque occurrence, de façon identique ou différente, 0, 1, 2, 3 ou 4;
q est 1, 2, 3, 4 ou 5.

2. Composés selon la revendication 1, **caractérisés en ce que** les indices n, de façon identique ou différente à chaque occurrence, sont 0 ou 1, de préférence 0.

3. Composés selon la revendication 1 ou 2, choisis parmi les structures des formules (2) à (7), 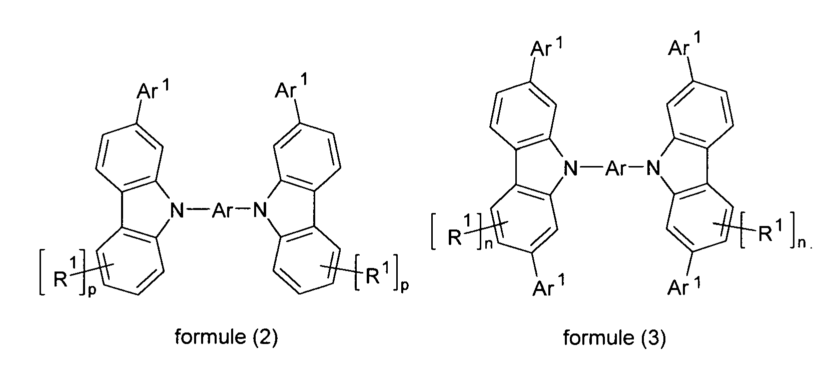 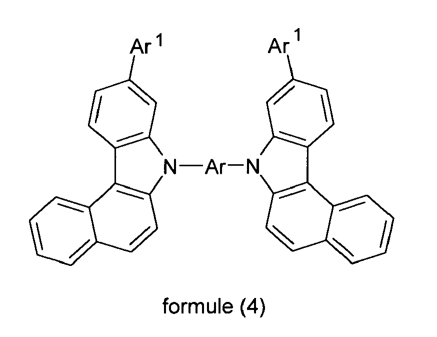 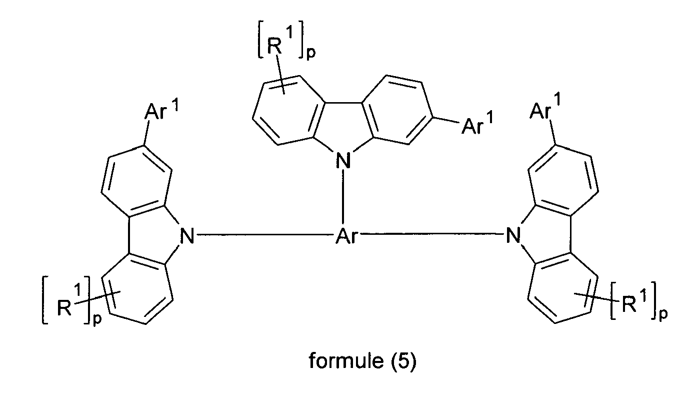 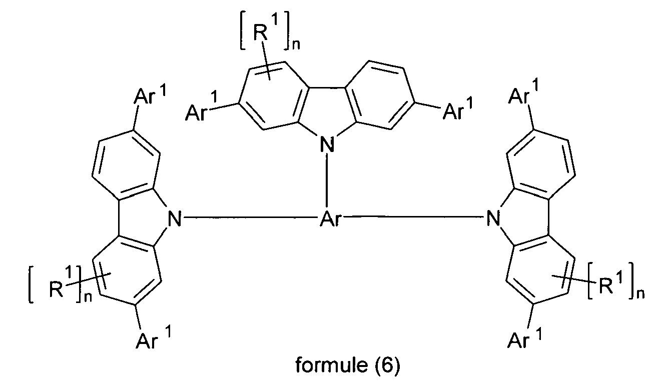 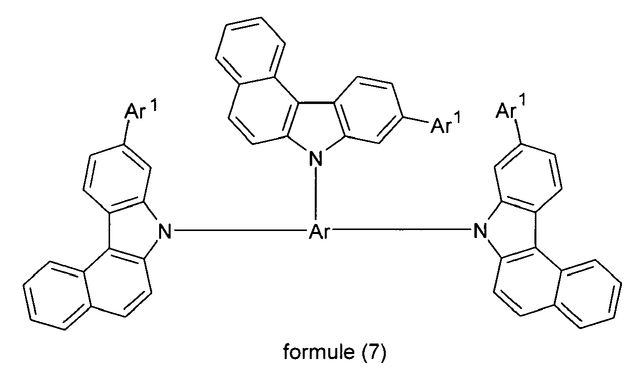 dans lesquelles les symboles et indices ont les significations indiquées dans la revendication 1.

4. Composés selon une ou plusieurs des revendications 1 à 3, **caractérisés en ce que** l'indice p, de façon identique ou différente à chaque occurrence, est 0, 1 ou 2, de préférence 0 ou 1.

5. Composés selon une ou plusieurs des revendications 1 à 4, **caractérisés en ce que** le substituant R¹ est lié dans la position 5 ou la position 7 du carbazole si l'indice p = 1, ou **en ce que** les substituants R¹ sont liés dans la position 5 et 7 du carbazole si l'indice p = 2.

6. Composés selon une ou plusieurs des revendications 1 à 5, **caractérisés en ce que** les groupes Ar et Ar¹ sont des systèmes de cycle aromatique formés à partir de groupes phényle et/ou naphtyle ou des systèmes liés de ce type, de groupes hétéroaromatiques n'ayant pas plus de deux cycles aromatiques ou hétéroaromatiques condensés ou des systèmes liés de ce type ou carbazole.

7. Composés selon une ou plusieurs des revendications 1 à 6, **caractérisés en ce que** le groupe Ar est choisi parmi 1,2-phénylène, 1,3-phénylène, 1,4-phénylène, 1,3,5-benzène, 3,3'-biphényle, 4,4'-biphényle, 1,3,5-triphénylbenzène, triphénylamine, 2,7-fluorénylène, chacun de ceux-ci peut être substitué par un ou plusieurs radicaux R¹, 2,7-spirobifluorénylène, qui peuvent être substitués par un ou plusieurs radicaux R¹, indénofluorénylène, qui peuvent être substitués par un ou plusieurs radicaux R¹, 4,4"'-(1,1':2',1",2",1"'-quaterphényle), 4,4'-(2,2'-diméthylbiphényle), 4,4'-(1,1'-binaphtyle), 4,4'-stilbényle ou dihydrophénanthrényle, chacun de ceux-ci peut être substitué par un ou plusieurs radicaux R¹.

8. Composés selon une ou plusieurs des revendications 1 à 7, **caractérisés en ce que** les groupes Ar¹ sont choisis, de façon identique ou différente, parmi phényle, 1-naphtyle, 2-naphtyle, triphénylamine, 2-carbazolyle, 3-carbazolyle, 9-carbazolyle, naphtyldiphénylamine ou dinaphtylphénylamine, chacun de ceux-ci peut être substitué par un ou plusieurs radicaux R.

9. Composés selon une ou plusieurs des revendications 1 à 8, **caractérisés en ce que** le symbole R représente, de façon identique ou différente à chaque occurrence, H, N(Ar²)₂, un groupe alkyle en chaîne droite ayant 1 à 5 atomes de C ou un groupe alkyle ramifié ayant 3 à 5 atomes de C, dans lequel dans chaque cas un ou plusieurs groupes CH₂ non adjacents peuvent être remplacés par -R²C=CR²-ou -O- et dans lequel un ou plusieurs atomes de H peuvent être remplacés par F, ou un groupe aryle ayant 6 à 16 atomes de C ou un groupe hétéroaryle ayant 2 à 16 atomes de C ou un groupe spirobifluorène, chacun de ceux-ci peut être substitué par un ou plusieurs radicaux R², ou une combinaison de deux de ces systèmes.

10. Composés selon une ou plusieurs des revendications 1 à 9, **caractérisés en ce qu'**ils sont des composés symétriques dans lesquels tous les symboles Ar¹ sont identiques.

11. Procédé pour la préparation de composés selon une ou plusieurs des revendications 1 à 10 par réaction d'un 4-aryl-2-nitro-1,1'-biphényle ou 4-hétéroaryl-2-nitro-1,1'-biphényle, dans lequel le groupe aryle ou hétéroaryle peut être substitué par un ou plusieurs radicaux R et le biphényle peut être substitué par un ou plusieurs radicaux R¹, avec un phosphite trialkyle, dans lequel les groupes alkyle, de façon identique ou différente à chaque occurrence, ont 1 à 10 atomes de C, pour donner le carbazole correspondant, ceci étant suivi par un couplage de Hartwig-Buchwald à un composé aromatique Ar qui comprend au moins deux groupes réactifs, qui sont de préférence choisis parmi chlore, brome, iode, triflate, tosylate ou OSO₂-R², dans lequel R² a la même signification que celle indiquée dans la revendication 1.

12. Utilisation de composés selon une ou plusieurs des revendications 1 à 10 dans des dispositifs électroniques organiques, en particulier dans des dispositifs électroluminescents organiques.

13. Dispositif électronique organique, en particulier dispositif électroluminescent organique (OLED, PLED), transistor à effet de champ organique (O-FET), transistor à film mince organique (O-TFT), transistor émetteur de lumière organique (O-LET), circuit intégré organique (O-IC), cellule solaire organique (O-SC), dispositif à désactivation de champ organique (O-FQD), cellule électrochimique émettrice de lumière (LEC), diode laser organique (O-laser) ou photorécepteur organique, comprenant au moins un composé selon une ou plusieurs des revendications 1 à 10 dans au moins une couche.

14. Dispositif électroluminescent organique selon la revendication 13, comprenant une cathode, une anode et au moins une couche émettrice et optionnellement d'autres couches, dans chaque cas choisies parmi une ou plusieurs couches d'injection de trous, couches de transport de trous, couches de blocage de trous, couches de transport d'électrons, couches d'injection d'électrons et/ou couches de génération de charges.

15. Dispositif électroluminescent organique selon la revendication 14, **caractérisé en ce que** le composé selon une ou plusieurs des revendications 1 à 10 est employé comme matrice pour des dopants phosphorescents.

16. Dispositif électroluminescent organique selon la revendication 15, **caractérisé en ce que** le composé selon une ou plusieurs des revendications 1 à 10 est employé en association avec une cétone aromatique, un oxyde de phosphine aromatique, un sulfoxyde aromatique ou un sulfone aromatique comme matrice pour des dopants phosphorescents.

17. Dispositif électroluminescent organique selon la revendication 15 ou 16, **caractérisé en ce que** le dopant phosphorescent comprend au moins un composé qui émet de la lumière lors d'une excitation appropriée et qui contient au moins un atome ayant un numéro atomique supérieur à 20, de préférence supérieur à 38 et inférieur à 84.

18. Dispositif électroluminescent organique selon une ou plusieurs des revendications 14 à 17, **caractérisé en ce que** le composé selon une ou plusieurs des revendications 1 à 10 est employé comme matériau de transport de trous ou comme matériau d'injection de trous, de préférence dans une couche de transport de trous ou dans une couche d'injection de trous.
